# EUROPEAN PATENT SPECIFICATION

(11) **EP 3 651 769 B1**
(45) Date of publication and mention of the grant of the patent: **05.01.2022**
(21) Application number: 18832869.4
(22) Date of filing: 12.07.2018
(51) Int. Cl.: A61K 9/00, A61K 9/08, A61K 9/20, A61K 9/48, A61K 31/277, A61K 31/4439, A61K 31/506

(54) **PROMOTING HAIR GROWTH AND TREATMENT OF HAIR LOSS OR EXCESSIVE HAIR SHEDDING**
FÖRDERUNG DES HAARWACHSTUMS UND BEHANDLUNG VON HAARAUSFALL ODER ÜBERMÄSSIGEM HAARLASSEN
STIMULATION DE LA POUSSE DES CHEVEUX ET TRAITEMENT DE LA CHUTE DE CHEVEUX OU DE L'ALOPÉCIE EXCESSIVE

(30) Priority: 12.07.2017 AU 2017902722
(43) Date of publication of application: 20.05.2020
(73) Proprietor: Samson Clinical Pty Ltd., Melbourne, Victoria 3000 (AU)
(72) Inventor: SINCLAIR, Rodney, East Melbourne, Victoria 3002 (AU)
(74) Representative: Vos, Derk
(86) International application number: PCT/AU2018/050719
(87) International publication number: WO 2019/010535

(56) References cited:
- WO-A1-2012/140252
- WO-A1-2016/065426
- WO-A2-99/40898
- WO-A2-2007/023396
- WO-A2-2010/036947
- WO-A2-2013/181487
- AU-B4- 2011 100 917
- GB-A- 2 314 019
- US-A- 4 997 643
- US-A1- 2007 166 362
- US-B1- 9 561 224

## Description

### Technical Field

The present invention relates to methods and compositions for treatment of excessive hair shedding or hair loss in subject or for promoting hair growth in a subject.

### Background

Hair follicles on the scalp do not continuously produce hair. They cycle through a growth stage that can last two or more years, then regress to a resting stage for up to three months before starting to grow a new hair fiber again. At any time on a healthy human scalp, about 80% to 90% of the hair follicles are growing hair. These active follicles are in what is called the anagen phase. That leaves up to 10% to 20% percent of scalp hair follicles in a resting state called telogen, when they don't produce any hair fiber. Changes in actual amount of hair fall occur in number of hair loss conditions including anagen effluvium, chemotherapy induced hair loss, acute and chronic telogen effluvium, alopecia areata, cicatricial alopecia, male pattern hair loss (MPHL) and female pattern hair loss (FPHL).

Men commonly complain of increased hair loss or hair shedding, especially after washing their hair. Changes in actual amount of hair fall occur in number of hair loss conditions including anagen effluvium, chemotherapy induced hair loss, acute and chronic telogen effluvium, alopecia areata, cicatricial alopecia and male pattern hair loss (MPHL).

Female pattern hair loss (FPHL) is the most common cause of hair loss encountered in clinical practice for women (Messenger et al. 2010). FPHL is a complex polygenic disorder characterised clinically by diffuse hair thinning over the mid frontal scalp and histologically by hair follicle miniaturization. The proportion of miniaturized follicles increases with the severity of hair loss (Messenger et al. 2006). FPHL adversely impacts quality of life and the prevalence of FPHL increases with age. In a population study of over 700 women, FPHL was found in 12% of women aged 20-29 and 57% of women aged > 80. Hair loss severity also increases with age.

One such condition that results in increased hair loss or excessive hair shedding is telogen effluvium (TE). This condition affects both men and women, occurring more commonly in women. TE is a non-scarring alopecia characterised by excessive shedding of telogen club hair diffusely from the scalp. It generally begins 8-12 weeks after a triggering event such as childbirth, major illness or complicated surgery and is resolves within 3-6 months. Once resolved, self-limiting telogen effluvium can be retrospectively diagnosed as acute telogen effluvium (Harrison S and Sinclair R, 2002). Telogen shedding that persists beyond 6 months is called chronic telogen effluvium (CTE) (Whiting, DA 1996).

Topical minoxidil has been suggested as a treatment for CTE, however results are variable and often disappointing. Application of a lotion to the scalp is also not desired by many individuals as it can result in hair looking oily which can interfere with compliance. In addition, high concentrations of topical minoxidil can have adverse effects on blood pressure due to the breakdown of minoxidil to minoxidil sulphate by the liver. There are currently no FDA or TGA treatments available for chronic telogen effluvium.

There is a requirement for new treatments for conditions of hair loss and excessive hair shedding, and for the promotion of hair growth (including increasing hair length and beard growth).

### Summary

The present invention relates to methods and compositions for the treatment of hair loss or excessive hair shedding in a subject or for promoting hair growth in a subject by administration of a dose of minoxidil absorbed by the oral mucosa or nasal mucosa. Such methods and compositions are advantageously fast acting compared to drugs which enter the blood stream via the lower gastrointestinal tract (which includes the stomach, small intestine, large intestine and rectum). Such compositions are easy to administer and likely to achieve increased patient compliance compared to traditional treatments i.e. such as tablets which are absorbed in the lower gastrointestinal tract and thus must be swallowed by patients and topical lotions which can leave hair looking oily or negatively impact on the look and feel of the hair. In addition, absorption through the oral mucosa or nasal mucosa allows drugs to by-pass first-pass metabolism in the liver.

In one aspect, the present invention provides a method of treating hair loss or excessive hair shedding in a subject or for promoting hair growth in a subject by administering to a subject an effective dose of minoxidil through the oral mucosa or nasal mucosa.

In an embodiment, the oral mucosa is selected from one or more of the sublingual mucosa, the buccal mucosa, the labial mucosa and/or the alveolar mucosa. In an embodiment, the oral mucosa is the sublingual mucosa. In an embodiment, the oral mucosa is the buccal mucosa. In an embodiment, the oral mucosa is the labial mucosa.

In one aspect, the present invention provides a method of treating hair loss or excessive hair shedding in a subject or for promoting hair growth in a subject by administering to a subject an orodispersive dose of minoxidil.

In an embodiment, the dose of minoxidil is in a form which disintegrates in the presence of saliva. In one embodiment, the dose of minoxidil is in a from which disintegrates in the presence of saliva and water.

In an embodiment, the dose of minoxidil results in a minoxidil blood concentration of about 0.25 ng/mL to about 10 ng/mL.

In an embodiment, the dose of minoxidil is within the range from about 0.05 mg to 3 mg, or from about 0.1 mg to 2.5 mg, or from about 0.15 mg to 2 mg, or from about 0.15 mg to 1.5 mg, or from about 0.15 mg to 1 mg, or from about 0.15 mg to 0.8 mg, or from about 0.15 mg to 0.5 mg, or from about 0.5 mg to 0.25 mg, or is about 0.1 mg, or is about 0.15 mg, or is about 0.24 mg, or is about 0.25 mg, or is about 0.45 mg, or is about 1.35 mg.

In an embodiment, the dose of minoxidil is about 0.45 mg.

In an embodiment, the dose of minoxidil is administered at least every 3 days, or at least every 2 days, or at least daily, or at least twice daily. In an embodiment, the dose of minoxidil is administered daily.

In an embodiment, the dose of minoxidil is in a form selected from a: strip, wafer, pellet, film, troche, tablet, lipid matrix tablet, capsule, pill, granule, pellet, powder, drop, spray and lozenge. In an embodiment, the dose of minoxidil is in a strip. In an embodiment, the dose of minoxidil is in a wafer. In an embodiment, the dose of minoxidil is in a pellet. In an embodiment, the dose of minoxidil is in a film.

In an embodiment, the present invention provides one or more of the dosage forms as described herein. In an embodiment, the present invention provides a package comprising one or more of the strips as described herein. In an embodiment, the present invention provides a package comprising one or more of the wafers as described herein. In an embodiment, the present invention provides a package comprising one or more of the films as described herein.

In an embodiment, the method further comprises administering a: aldosterone antagonist, 5α-reductase inhibitor, non-steroidal antiandrogen drug and/or a steroidal antiandrogen.

In an embodiment, the method further comprises administering spironolactone within the range of from about 10 mg to 500 mg, or from about 10 mg to 400 mg, or from about 10 mg to 300 mg, or from about 15 mg to 200 mg, or from about 15 mg to 150 mg, or from about 18 mg to 100 mg, or from about 20 mg to 80 mg, or from about 20 mg to 50 mg, or from about 22 mg to 40 mg, or from about 23 mg to 35 mg, or from about 23 mg to 30 mg, or is about 25 mg. In an embodiment, spironolactone is at a concentration of about 25 mg.

In an embodiment, the method further comprises administering a pharmaceutical dose of salt. In an embodiment, the pharmaceutical dose of salt can be sodium chloride. In an embodiment, the method further comprises administering sodium chloride in the range of from about 10 mg to 200 mg, from about 15 mg to 150 mg, or from about 15 mg to 125 mg, or from about 20 mg to 100 mg, or from about 25 mg to 80 mg, or from about 30 mg to 70 mg, or from about 40 mg to 60 mg, or from about 45 mg to 55 mg. In an embodiment, sodium chloride is administered at a concentration of at least 10 mg, or at least 15 mg, or at least 20 mg, or at least 25 mg, or at least 30 mg, or at least 35 mg, or at least 40 mg, or at least 45 mg, or at least 50 mg, or at least 100 mg, or at least 200 mg. In an embodiment, sodium chloride is administered at a concentration of about 50 mg. In an embodiment, sodium chloride is administered at a concentration of about 20 mg.

In an embodiment, the method additionally comprises administering one or more of: (i) finasteride within the range of from about 0.1 mg to 1 mg; (ii) dutasteride within the range of from about 0.01 mg to 1 mg; (iii) flutamide within the range of from about 10 mg to 500 mg; (iv) cyproterone acetate within the range of from about 1 mg to 100 mg; (v) bicalutamide within the range of from about 1 mg to 100 mg; (vi) enzalutamide within the range of from about 1 mg to 100 mg; (vii) nilutamide within the range of from about 1 mg to 100 mg; (viii) drosperidone within the range of from about 0.1 mg to 10 mg; (ix) apalutamide within the range of from about 1 mg to 100 mg; and/or (x) buseralin within the range of from about 0.1 mg to 10 mg.

In an embodiment, the method further comprises administering an excipient. In an embodiment, the excipient is selected from one or more of: starch, corn starch, colloidal silicon dioxide, lactose, magnesium stearate, microcristaline cellulose, anhydrous lactose, docusate sodium, magnesium stearate, microcrystalline cellulose, sodium benzoate and sodium starch glycolate.

In an embodiment, the method further comprises administering zinc. In an embodiment, the method further comprises administering zinc within the range of from about 0.1 mg to 100 mg, or from about 0.1 mg to 75 mg, or from about 0.1 mg to 50 mg, or from about 0.1 mg to 20 mg, or from about 1 mg to 15 mg, or from about 2.5 mg to 15 mg, or from about 5 mg to 13 mg, or from about 8 mg to 12 mg, or from about 10 mg to 12 mg daily. In an embodiment, the zinc concentration is about 5 mg daily. In an embodiment, the zinc concentration is about 8 mg daily. In an embodiment, the zinc concentration is about 12 mg daily.

In an embodiment, the method further comprises administering selenium. In an embodiment, the method further comprises administering selenium within the range of from about 10 µg to 200 µg, or from about 15 µg to 150 µg, or from about 15 µg to 125 µg, or from about 20 µg to 100 µg, or from about 25 µg to 80 µg, or from about 30 µg to 70 µg, or from about 40 µg to 60 µg, or from about 45 µg to 55 µg daily. In an embodiment, selenium is administered at a concentration of about 50 µg daily. In an embodiment, selenium is administered at a concentration of about 20 µg daily.

In an embodiment, the method further comprises administering caffeine. In an embodiment, the method further comprises administering caffeine within the range of from about 50 mg to 250 mg, or from about 60 mg to 240 mg, or from about 80 mg to 220 mg, or from about 100 mg to 200 mg, or from about 100 mg to 150 mg daily.

In an embodiment, the method further comprises administering liquorice. In an embodiment, the method further comprises administering liquorice within the range of from about 50 mg to 250 mg, or from about 60 mg to 240 mg, or from about 80 mg to 220 mg, or from about 100 mg to 200 mg, or from about 100 mg to 150 mg daily.

In an embodiment, the method further comprises administering a vitamin, wherein the vitamin is selection from: vitamin A, vitamin B, vitamin C and vitamin D.

In an embodiment, the method further comprises administering an amino acid, wherein the amino acid is selected from tyrosine, methionine, thymine, arginine, cysteine, lysine and cysteine.

In an embodiment, the hair loss or excessive hair shedding is the result of one or more of the following; hair follicle miniaturization, alopecia areata, androgenetic alopecia, telogen effluvium, anagen effluvium, chemotherapy induced hair loss, male pattern baldness, female pattern baldness, monilethrix, thyroid problems, anaemia, polycystic ovary syndrome, cicatricial alopecia (lichen planopilaris, discoid lupus erythematosus, folliculitis decalvans), congenital hypotrichosis, loose anagen hair syndrome, hypotrichosis and malnutrition. In an embodiment, anagen effluvium includes alopecia areata, loose anagen hair syndrome and drug induced hair loss. In an embodiment, the hair loss or excessive hair shedding is the result of male pattern baldness. In an embodiment, the hair loss or excessive hair shedding is the result of female pattern baldness. In an embodiment, the hair loss or excessive hair shedding is the result of telogen effluvium.

In an embodiment, promoting hair growth comprises promoting beard growth in a subject. In an embodiment, prompting hair growth comprises increasing hair length.

In one aspect, the present invention provides an orodispersible composition for treating hair loss or excessive hair shedding in a subject or promoting hair growth in a subject comprising; (i) minoxidil within the range of from about 0.05 mg to 3 mg; (ii) minoxidil at a concentration of about 0.1 mg; (iii) minoxidil at a concentration of about 0.15 mg; (iv) minoxidil at a concentration of about 0.24 mg; (v) minoxidil at a concentration of about 0.25 mg; (vi) minoxidil at a concentration of about 0.45 mg; (vi) minoxidil at a concentration of about 1.35 mg; (vii) minoxidil within the range of from about 0.05 mg to 3 mg and spironolactone within the range of from about 10 mg to 500 mg; or (viii) minoxidil within the range of from about 0.15 mg to 1.35 mg and spironolactone within the range of from about 10 mg to 500 mg.

In an embodiment, the composition is in a form selected from a: strip, wafer, film, troche, tablet (including a mini-tablet), lipid matrix tablet, capsule, pill, granule, pellet, powder, drop, spray and lozenge. In an embodiment, the spray is a powder. In an embodiment, the composition is in a form selected from: strip, wafer, pellet and film. In an embodiment, the composition is a strip. In an embodiment, the composition is a wafer. In an embodiment, the composition is a pellet. In an embodiment, the composition is a film. In an embodiment, the composition is a troche. In an embodiment, the composition is a tablet. In an embodiment, the composition it a capsule. In an embodiment, the composition is a pill. In an embodiment, the composition is a powder. In an embodiment, the composition is a drop. In an embodiment, the composition is a spray. In an embodiment, the composition is a lozenge.

In an aspect, the present invention provides a composition for treating hair loss or excessive hair shedding or promoting hair growth in a subject via administration to the oral mucosa or nasal mucosa comprising; (i) minoxidil within the range of from about 0.05 mg to 3 mg; (ii) minoxidil at a concentration of about 0.1 mg; (iii) minoxidil at a concentration of about 0.15 mg; (iv) minoxidil at a concentration of about 0.24 mg; (v) minoxidil at a concentration of about 0.25 mg; (vi) minoxidil at a concentration of about 0.45 mg; (vi) minoxidil at a concentration of about 1.35 mg; (vii) minoxidil within the range of from about 0.05 mg to 3 mg and spironolactone within the range of from about 10 mg to 500 mg; or (viii) minoxidil within the range of from about 0.15 mg to 1.35 mg and spironolactone within the range of from about 10 mg to 500 mg; wherein the composition is in the form of a strip, wafer, pellet or film.

In an embodiment, the composition further comprises a disintegration agent which aids disintegration of the composition in the presence of saliva.

In an embodiment, the composition further comprises an agent which can be acted upon by an enzyme in saliva to facilitate disintegration.

In an embodiment, the composition additionally comprises a taste modifying agent.

In an aspect, the present invention provides a composition for treating hair loss or excessive hair shedding in a subject or promoting hair growth in a subject via administration to the nasal mucosa comprising; (i) minoxidil within the range of from about 0.05 mg to 3 mg; (ii) minoxidil at a concentration of about 0.1 mg; (iii) minoxidil at a concentration of about 0.15 mg; (iv) minoxidil at a concentration of about 0.24 mg; (v) minoxidil at a concentration of about 0.25 mg; (vi) minoxidil at a concentration of about 0.45 mg; (vi) minoxidil at a concentration of about 1.35 mg; (vii) minoxidil within the range of from about 0.05 mg to 3 mg and spironolactone within the range of from about 10 mg to 500 mg; or (viii) minoxidil within the range of from about 0.15 mg to 1.35 mg and spironolactone within the range of from about 10 mg to 500 mg, wherein the composition is in the form of a drop or spray.

In an embodiment, the composition additionally comprises one or more of: (i) sodium chloride at a concentration of from about 10 to 200 mg; (ii) sodium chloride at a concentration of about 50 mg; (iii) zinc at a concentration of about 0.1 to 100 mg; (iv) zinc at a concentration of about 8 mg; (v) selenium at a concentration of 20 µg to 200 µg; (vi) caffeine at a concentration of about 50 mg to 250 mg; (vii) liquorice at a concentration of about 50 mg to 250 mg; (viii) at least one vitamin; and/or (ix) at least one amino acid.

In an embodiment, the composition additionally comprises one or more of: (i) finasteride within the range of from about 0.1 mg to 1 mg; (ii) dutasteride within the range of from about 0.01 mg to 1 mg; (iii) flutamide within the range of from about 10 mg to 500 mg; (iv) spironolactone within the range of from about 10 mg to 500 mg; (v) cyproterone acetate within the range of from about 1 mg to 100 mg; (vi) bicalutamide within the range of from about 10 mg to 500 mg; (vii) enzalutamide within the range of from about 1 mg to 100 mg; (viii) nilutamide within the range of from about 1 mg to 100 mg; (ix) drosperidone within the range of from about 0.1 mg to 10 mg; (x) apalutamide within the range of from about 1 mg to 100 mg; and/or (xi) buseralin within the range of from about 0.1 mg to 10 mg.

In an embodiment, the hair loss or excessive hair shedding is the result of one or more of the following; hair follicle miniaturization, alopecia areata, androgenetic alopecia, telogen effluvium (chronic and acute), anagen effluvium, chemotherapy induced hair loss, male pattern baldness, female pattern baldness, thyroid problems, monilethrix anaemia, polycystic ovary syndrome, cicatricial alopecia (lichen planopilaris, discoid lupus erythematosus, folliculitis decalvans), congenital hypotrichosis, hypotrichosis and malnutrition. In an embodiment, the condition is male pattern baldness. In an embodiment, the condition is female pattern baldness. In an embodiment, the condition is androgenic alopecia. In an embodiment, the condition is telogen effluvium. In an embodiment, the condition is chronic telogen effluvium. In an embodiment, the condition is acute telogen effluvium.

In an embodiment, promoting hair growth comprises promoting beard growth in a subject. In an embodiment, prompting hair growth comprises increasing hair length. As described herein according to any example minoxidil may be additionally administered with one or more other treatments for hair loss or excessive hair shedding. In an embodiment, other treatments for hair loss or excessive hair shedding include treatments which are administered orally, intravenously and/or topically. In an embodiment, minoxidil is additionally administered with an oral antiandrogen. Exemplary treatments include: finasteride (propecia), dutasteride (avodart), flutamide, spironolactone (aldactone), cimetidine (tagamet), cyproterone acetate, bicalutamide, enzalutamide, nilutamide, apalutamide, buserelin, trans retinoic acid, oral contraceptives such as low dose androgen index birth control pills, estrogen and/or progesterone.

In an embodiment, the dose of minoxidil and compositions as described herein do not comprise a histone deacetylase inhibitor 4 (HDAC4) inhibitor.

As described herein according to any example, the subject has a condition characterized by hair loss or excessive hair shedding. As described herein according to any example, the subject has a condition characterized by hair follicle miniaturization. As described herein according to any example, the hair loss or excessive hair shedding is the result of a genetic condition. As described herein according to any example, the hair loss or excessive hair shedding is the result of environmental factors. Exemplary conditions include alopecia areata, androgenetic alopecia, telogen effluvium, anagen effluvium (associated which chemotherapy), male pattern baldness, female pattern baldness, monilethrix, thyroid problems (e.g. disease characterized by hypothyroidism and hyperthyroidism), scale infections, anaemia, polycystic ovary syndrome, cicatricial alopecia (lichen planopilaris, discoid lupus erythematosus, folliculitis decalvans), congenital hypotrichosis, hypotrichosis and malnutrition. In an embodiment, the condition is androgenic alopecia.

As described herein according to any example minoxidil may be administered before, during or after chemotherapy treatment for the treatment of chemotherapy induced hair loss or excessive hair shedding or promoting hair growth after chemotherapy treatment. As described herein according to any example the subject is a mammal. As described herein according to any example the subject is human. In an embodiment, the subject may be male. In an embodiment, the subject may be female.

### Brief Description of Drawings

**Figure 1****:** Shows sublingual minoxidil dosage from comprising 5 mg of minoxidil.
**Figure 2****:** Shows scalp section from Patient 1 (A) at baseline and (B) after 12 weeks of treatment with sublingual minoxidil in troche form. At baseline the total hair count was 108 (terminal 100; vellus 8) at 12 weeks the total hair count was 129 (terminal 123; vellus 6) an increase of 21 hairs (19.4%).
**Figure 3****:** Shows the part line of Patient 1 (A) at baseline and (B) after 12 weeks of treatment with sublingual minoxidil in troche form.
**Figure 4****:** Shows scalp section from Patient 2 (A) at baseline (B) and after 12 weeks of treatment with sublingual minoxidil in troche form. At baseline the total hair count was 167 (terminal 151; vellus 16) at 12 weeks the total hair count was 199 (terminal 186; vellus 13) an increase of 32 hairs (19.2%).
**Figure 5****:** Shows the part line of Patient 2 (A) at baseline (B) and after 12 weeks of treatment with sublingual minoxidil in troche form.
**Figure 6****:** Shows scalp section from Patient 3 (A) at baseline and (B) after 12 weeks of treatment with sublingual minoxidil in troche form. At baseline the total hair count was 61 (terminal 50; vellus 11) at 12 weeks the total hair count was 84 (terminal 71; vellus 13) an increase of 23 hairs (37.7%).
**Figure 7****:** Shows the part line of Patient 3 (A) at baseline and (B) after 12 weeks of treatment with sublingual minoxidil in troche form.

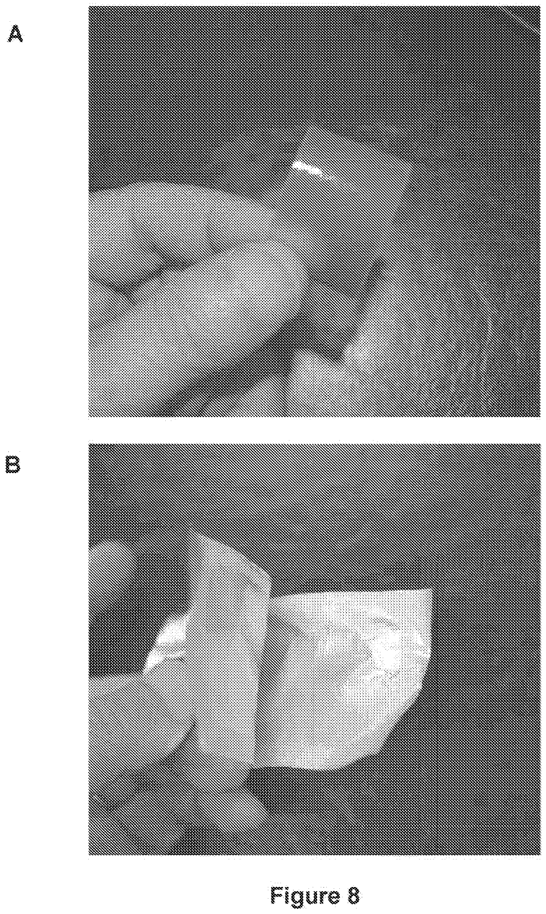

**Figure 8****:** Shows an embodiment of the invention, (A) an orodispersible film and (B) a individually packaged orodispersible film.
**Figure 9****:** Shows a global photograph from a patient at baseline and after 8 weeks of daily treatment with 0.45 mg sublingual minoxidil.
**Figure 10****:** Shows a global photograph from a patient at baseline and after 8 weeks of daily treatment with 0.45 mg sublingual minoxidil.
**Figure 11****:** Shows a global photograph from a patient at baseline and after 8 weeks of daily treatment with 0.45 mg sublingual minoxidil.
**Figure 12****:** Shows macrophotographs from a female patient at baseline and after 8 weeks of daily treatment with 0.45 mg sublingual minoxidil.
**Figure 13****:** Shows macrophotographs from a male patient at baseline and after 8 weeks of daily treatment with 0.45 mg sublingual minoxidil.
**Figure 14****:** Shows macrophotographs from a male patient at baseline and after 8 weeks of daily treatment with 0.45 mg sublingual minoxidil.

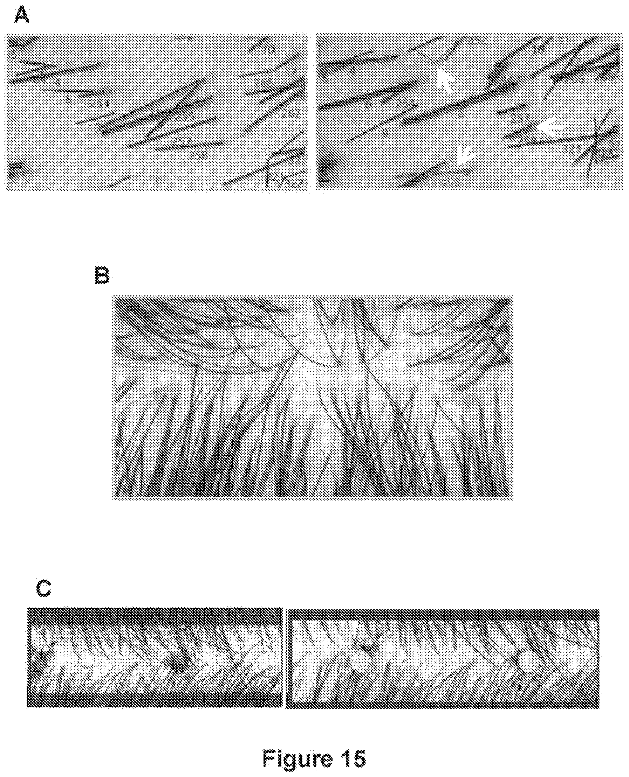

**Figure 15:** (A) Shows an example of clipped hair Hair to Hair (H2H) matched phototrichogram in images taken at an initial time point (left) and a subsequent time point (right). Individual hair strands are identified and numbered. H2H technology allows identification of the same hair strands in a region of interest at a different time point. Arrows in the right image indicate newly identified hair strands not present in the initial image (left). A proprietary spot template is used to assist in determining the points to be marked on the scalp for macrophotography. Pairs of spots (x3) will be identified and marked on the patient's scalp: 1 pair for phototrichogram and 2 for phototrichoscopy spots. An example of the spots is shown in (B). The spots are tattooed onto the patients scalp. Virtual tattoo technology as shown in (C) is used to ensure pre-marked spots are aligned and images are identically orientated.

### Description of Embodiments

As described herein, "minoxidil" also known as "2,4-Diamino-6-piperidinopyrimidine 3-oxide" or "2,4-Pyrimidinediamine, 6-(1-piperidinyl)-, 3-oxide" or "2,6-Diamino-4-piperidinopyrimidin-1-oxid" is a piperidinopyrimidine derivative and a potent vasodilator (CAS ID: 38304-91-5). The term "minoxidil" is used in broad sense to include not only "minoxidil" *per se* but also its pharmaceutically acceptable derivatives thereof. Suitable derivatives include pharmaceutically acceptable salts, pharmaceutically acceptable solvates, pharmaceutically acceptable hydrates, pharmaceutically acceptable sulfates, pharmaceutically acceptable anhydrates, pharmaceutically acceptable enantiomers, pharmaceutically acceptable esters, pharmaceutically acceptable isomers, pharmaceutically acceptable polymorphs, pharmaceutically acceptable prodrugs, pharmaceutically acceptable tautomers, pharmaceutically acceptable complexes etc.

In an embodiment, minoxidil as described herein, is converted in a subject to minoxidil sulphate. In an embodiment, minoxidil as described herein is converted to minoxidil sulphate in a hair follicle.

As described herein, "spironolactone" is an aldosterone antagonist and has been used as a potassium-sparing diuretic for over 50 years (CAS ID: 52-01-7). It is structurally a steroid, with basic steroid nuclei with four rings.

As described herein, "finasteride", also referred to as "propecia", is a type II 5α-reductase inhibitor, it acts by inhibiting the activity of 5α-reductase, an enzyme that converts testosterone to dihydrotestosterone (CAS ID: 98319-26-7). It is a synthetic drug for the treatment of benign prostatic hyperplasia and male pattern baldness and can be administered orally.

As described herein, "dutasteride" is a 5-α reductase inhibitor that inhibits conversion of testosterone to dihydrotestosterone (CAS ID: 164656-23-9).

As described herein, "flutamide" is a non-steroidal antiandrogen drug (CAS ID: 13311-84-7).

As described herein, "cyproterone" is a steroidal antiandrogen (CAS ID: 2098-66-0).

As described herein, "bicalutamide" is a non-steroidal antiandrogen drug (CAS ID: 90357-06-5).

As described herein, "enzolutamide" is a non-steroidal antiandrogen drug (CAS ID: 90357-06-5).

As described herein, "nilutamide" is a non-steroidal antiandrogen drug (CAS ID: 90357-06-5).

As described herein, "apalutamide" is a non-steroidal antiandrogen drug (CAS ID: 90357-06-5).

As described herein, "buserilin" is a non-steroidal antiandrogen drug (CAS ID: 90357-06-5).

As described herein, "saw palmetto" is a non-steroidal antiandrogen drug (CAS ID: 90357-06-5).

As described herein, "azeleic acid" is a non-steroidal antiandrogen drug (CAS ID: 90357-06-5).

As described herein, "buserilin" is a non-steroidal antiandrogen drug (CAS ID: 90357-06-5).

As used herein the term "subject" refers to a mammal, particularly a human. In an embodiment the subject, is male. In an embodiment the subject, is female.

As used herein, the terms "treating" or "treatment" of hair loss or hair shedding means: (1) preventing or delaying the appearance of clinical symptoms of the state, disorder or condition developing in a mammal that may be afflicted with or predisposed to the state, disorder or condition but does not yet experience or display clinical or subclinical symptoms of the state, disorder or condition, (2) inhibiting the state, disorder or condition, i.e., arresting or reducing the development of the disease or at least one clinical or subclinical symptom thereof, or (3) relieving the disease, i.e., causing regression of the state, disorder or condition or at least one of its clinical or subclinical symptoms.

As used herein, the terms "promoting" or "promotion" of hair growth refers to inducing or supporting hair growth. In an embodiment, the present invention promotes beard growth in a subject. In an embodiment, promoting hair growth increases the number of hair follicles in the anagen hair growth phase. In an embodiment, promoting hair growth comprises increasing the length of the anagen hair growth phase. In an embodiment, promoting hair growth comprises increasing the initiation of the anagen hair growth phase. In an embodiment, promoting hair growth decreases the length of the telogen hair growth phase. In an embodiment, promoting hair growth decreases the length of the catgen hair growth phase. In an embodiment, promoting hair growth decreases the length of the kenogen hair growth phase. In an embodiment, promoting hair growth comprises increasing hair length. In an embodiment, promoting hair growth comprises increasing the diameter of hair fibres. In an embodiment, promoting hair growth comprises increasing the number of hairs in a hair follicle. In an embodiment, promoting hair growth increases the number of frontal scalp terminal hairs.

The methods and compositions as described herein are relevant to the treatment of "hair loss". One particular form of "hair loss" is "hair shedding" described as where hair falls out from skin areas where it is usually present, such as the scalp. Hair shedding can be described as either normal levels of hair shedding or excessive levels of hair shedding. Excessive hair loss or hair shedding may be a consequence of one of the following conditions: alopecia areata, androgenetic alopecia, telogen effluvium (chronic and acute), anagen effluvium, chemotherapy induced hair loss, male pattern baldness, female pattern baldness, thyroid problems, monilethrix, anaemia, congenital hypotrichosis, hypotrichosis, short anagen syndrome, loose anagen syndrome, drug induced and chemotherapy induced hair loss, cicatricial alopecia (lichen planopilaris, discoid lupus erythematosus, folliculitis decalvans), polycystic ovary syndrome, cicatricial alopecia (lichen planopilaris, discoid lupus erythematosus, folliculitis decalvans), congenital hypotrichosis, hypotrichosis or malnutrition. In an embodiment, the condition is male pattern baldness. In an embodiment, the condition is androgenic alopecia. In an embodiment, the condition is telogen effluvium. In an embodiment, the condition is chronic telogen effluvium. In an embodiment, the condition is acute telogen effluvium. In an embodiment, the subject is female. In an embodiment, the subject is male.

In an embodiment, the methods and compositions as described herein reduce chemotherapy induced hair loss or excessive hair shedding and/or increase the rate of recovery from chemotherapy induced hair loss or excessive hair shedding. In an embodiment, the methods and compositions as described herein reduce the relapse of alopecia areata and/or increase the rate of recovery from alopecia areata.

As described herein, the "oral mucosa" refers to the mucous membrane lining the inside the oral cavity, which includes the sublingual mucosa, the buccal mucosa, the labial mucosa and/or the alveolar mucosa. A person skilled in the art would appreciate that the methods and compositions as described herein relate to a dose of minoxidil or composition comprising minoxidil that can enter the blood stream by crossing the oral mucosa. In contrast to doses which are absorbed in the intestines, doses absorbed in the mouth/oral cavity by-pass first-pass metabolism in the liver.

In an embodiment, the methods and compositions as described herein relate to a dose of minoxidil or composition comprising minoxidil wherein at least 30%, or at least 40%, or at least 50%, or at least 60%, or at least 70% or at least 80%, or at least 90% enters the blood stream by crossing the oral mucosa. In an embodiment, at least 60% enters the blood stream by crossing the oral mucosa. In an embodiment, at least 70% enters the blood stream by crossing the oral mucosa.

In contrast, many orally administered drugs are pills or tablets formulated for oral administration (are swallowed) and are absorbed in the intestines. For example 95% of an orally administered dose of minoxidil as approved by the Therapeutic Goods Administration (TGA) for adults with severe refractory hypertension is absorbed gastrointestinally.

As used herein, the term "orodispersible" refers to a dose or dosage form that dissolves, disintegrates and/or disperses in the mouth/oral cavity allowing for absorption in the mouth/oral cavity. Such dosage forms may also be referred to as "mouth dissolving" dosage forms. In an embodiment, at least 30%, or at least 40%, or at least 50%, or at least 60%, or at least 70%, or at least 80% or at least 90% of the orodispersible dose is absorbed in the mouth/oral cavity. In an embodiment, at least 60% of the orodispersible dose is absorbed in the mouth/oral cavity. In an embodiment, at least 70% of the orodispersible dose is absorbed in the mouth/oral cavity.

As used herein, the term "sublingual" or "sublingually" refers to the pharmacological route of administration wherein a desired substance diffuses, is actively transported and/or endocytosed into the blood through tissues under the tongue. The tissue under the tongue contains a large number of capillaries, once in the capillaries the substance enters the venous circulation. In a preferred example, the dose or dosage form as described herein is a sublingual dose wherein at least 30%, or at least 40%, or at least 50%, or at least 60%, or at least 70%, or at least 80% or at least 90% of a sublingual dose is absorbed sublingually. In an embodiment, at least 60% of a sublingual dose is absorbed sublingually. In an embodiment, at least 70% of a sublingual dose is absorbed sublingually

As described herein, the "nasal mucosa" refers to the mucous membrane lining the nasal passage. A person skilled in the art would appreciate that the methods and compositions as described herein relate to a dose of minoxidil or composition comprising minoxidil that can enter the blood stream by crossing the nasal mucosa.

Minoxidil may be absorbed across the oral mucosa or nasal mucosa by passive diffusion, active or carrier-mediated transport and/or endocytosis.

A person skilled in the art will appreciate that the dose of minoxidil or composition comprising minoxidil as described herein is formulated for absorption across the oral and/or nasal mucosa. Absorption across the oral mucosa may include absorption across one or more of the sublingual mucosa, the buccal mucosa, the labial mucosa and/or the alveolar mucosa. In a preferred embodiment, the dose of minoxidil or the composition comprising minoxidil is formulated for absorption across the sublingual mucosa. In a preferred embodiment, the dose of minoxidil or the composition comprising minoxidil is formulated for absorption across the buccal mucosa.

A person skilled in the art will appreciate that the dose of minoxidil or composition comprising minoxidil may be formulated in any form that allows minoxidil to cross the oral mucous membrane, such forms include, but are not limited to a; strip, wafer, film, troche, lipid matrix tablet, tablet (including a mini-tablet), capsule, pill, granule, pellet, powder, drop, spray and lozenge. In an embodiment, the spray is a powder. In an embodiment, the powder is packaged in a sachet. In an embodiment the dose of minoxidil or composition comprising minoxidil is formulated as a strip, wafer, pellet or film which disintegrates when placed under the tongue. In an embodiment the dose of minoxidil or composition comprising minoxidil is formulated as an "orodispersible strip", "orodispersible wafer" or an "orodispersible film". In an embodiment, the strip, wafer or film disperses/disintegrates sublingually. In an embodiment, the film may be selected from a flash release, mucoadhesive melt-away or a mucoadhesive sustained release film for example as described in Nagaraju et al. (2013). In an embodiment, the dose of minoxidil or composition comprising minoxidil is formulated as a buccal lozenge. In an embodiment, the dose of minoxidil or composition comprising minoxidil is formulated in a spray. In an embodiment, the spray can be applied to the buccal mucosa and/or the sublingual mucosa. In an embodiment, the dose of minoxidil or composition comprising minoxidil is not a nanoparticle composition. In an embodiment, the dose of minoxidil or composition comprising minoxidil is not a liquid. In an embodiment, the dose of minoxidil or composition comprising minoxidil is not a foam.

The dose of minoxidil or composition comprising minoxidil may be formulated for rapid disintegration to ensure minoxidil is absorbed in the oral cavity. In an embodiment, such doses or compositions will be formulated to disintegrate in the presence of saliva and/or water. In an embodiment, such formulations may comprise a disintegration agent which aids disintegration of the dose or composition in the presence of saliva and/or the presence of water.

In an embodiment, the dose of minoxidil results in a minoxidil blood concentration of about 0.25 ng/mL to about 10 ng/mL. In an embodiment, the dose of minoxidil results in a minoxidil blood concentration of about 0.25 ng/mL to about 8 ng/mL. In an embodiment, the dose of minoxidil results in a minoxidil blood concentration of about 0.25 ng/mL to about 7 ng/mL. In an embodiment, the dose of minoxidil results in a minoxidil blood concentration of about 0.25 ng/mL to about 6 ng/mL. In an embodiment, the dose of minoxidil results in a minoxidil blood concentration of about 0.25 ng/mL to about 5 ng/mL. In an embodiment, the dose of minoxidil results in a minoxidil blood concentration of about 0.25 ng/mL to about 4 ng/mL. In an embodiment, the blood concentration is the plasma concentration. In an embodiment, the blood concentration is the serum concentration. The blood minoxidil concentration may be measured by any method know to a person skilled in the art, including for example, LC MS/MS analysis.

In an embodiment, the dose of minoxidil or composition comprising minoxidil is stable at between about 0°C to about 40°C.

As used herein the "disintegrating agent", "disintegration agent" or "disintegrant/s" refers to an agent added to the dose or compositions that facilitates disintegration/dispersion of the formulation in the oral cavity and/or nasal cavity and includes superdisintegrating agents and effervescent agents. Disintegrants may act by water wicking, capillary action, swelling, deformation, repulsion (e.g. release of gasses), and heat of wetting.

Examples of disintegrating agents can be found in Gad et al. (2008) and Rowe et al. (2009) and include for example, but are not limited to, starch, modified starches, crosslinked starches, crosslinked alginic acid, modified cellulose and cross-linked povidone, microcrystalline cellulose, sodium starch glycollate (Primojel, Explotab), cassia fistula gum, crospovidone, croscarmellose sodium, alginic acid, sodium alginate, starch USP, starch 1500, avicel, solka floc, alginic acid, sodium alginate, polyplasdone, amberlite, methyl cellulose, AC-Di-Sol, carbon dioxide, lepidum sativum, locust bean gum, nymce ZSX, primellose, solutab, vivasol crosspovidone, crosspovidon M, kollidon, polyplasdone, plantagoovata husk, plantago ovate mucilage, cetric acid, satialgine, soy polysaccharides, sodium bicarbonate, sodium starch glycolate, treated agar, emcosoy, and calcium silicate.

Disintegrating agents that are particularly suitable for use in orodispersive films are described in Nagaraju et al. (2013).

In an embodiment, the disintegrating agent swells at least 2 fold in under 10 seconds, or at least 3 fold in under 10 seconds, or at least 4 fold in under 10 seconds, or at least 5 fold in under 10 seconds, or at least 6 fold in under 10 seconds, or at least 7 fold in under 10 seconds, or at least 8 fold in under 10 seconds. In an embodiment, the disintegrating agent swells at least 2 fold in under 30 seconds, or at least 3 fold in under 30 seconds, or at least 4 fold in under 30 seconds, or at least 5 fold in under 30 seconds, or at least 6 fold in under 30 seconds, or at least 7 fold in under 30 seconds, or at least 8 fold in under 30 seconds, or at least 9 fold in under 30 seconds, or at least 10 fold in under 30 seconds, or at least 11 fold in under 30 seconds, or at least 12 fold in under 30 seconds.

In an embodiment, the dose or composition is formulated so that an oral disintegrating enzyme facilitates disintegration in the oral cavity. In an embodiment, the oral disintegrating enzyme is amylase (which acts upon starch), protease (which acts upon gelatin), cellulase (which acts upon cellulose and/or its derivatives) and/or invertase (which acts upon sucrose).

In an embodiment, the dose of minoxidil or composition comprising minoxidil is formulated to disintegrate/disperse within 2 minutes, or within 1 minute, or within 50 seconds, or within 40 seconds, or within 30 seconds, or within 20 seconds, or within 10 seconds of being placed in the mouth. Disintegration rates can be assessed by any method known to a person skilled in the art.

In an embodiment, the dose of minoxidil or composition comprising minoxidil is formulated to disintegrate/disperse within 2 minutes, or within 1 minute, or within 50 seconds, or within 40 seconds, or within 30 seconds, or within 20 seconds, or within 10 seconds of being placed under the tongue. Disintegration rates can be assessed by any method known to a person skilled in the art.

In an embodiment, the dose of minoxidil or composition comprising minoxidil may comprise an agent which aids adherence of minoxidil to the oral or nasal mucosa to facilitate absorption.

In an embodiment, the dose of minoxidil or composition comprising minoxidil may comprise a taste modifying agent to improve the taste of the dose or composition for the subject. Taste modifying agents include sweeteners and flavouring agents. Examples of taste modifying agents can be found in Gad et al. (2008), Rowe et al. (2009) and Nagaraju et al. (2013) and include, for example mannitol, aspartame, sucrose, dextrose, fructose, glucose, maltose, neotame, alitame, saccharin and sorbitol.

The dose of minoxidil or composition comprising minoxidil may be formulated in any form that allows minoxidil to cross the nasal mucous membrane, such forms include, but are not limited to a; strip, wafer, pellet, film, granule, powder, drop, and spray/mist. In a preferred embodiment the dose of minoxidil or composition comprising minoxidil is formulated as a spray/mist which is administered to the nasal cavity.

A person skilled in the art will appreciate that such dosage forms or compositions may be prepared by any method known to a person skilled in the art and can include, for example: freeze-drying or lyophilisation, sublimation, spray drying, moulding, mass extrusion, direct compression, melt granulation, effervescent method, 3D printing, ink-jet technology (for application of minoxidil to strips, wafers and films). Examples of such methods can be found in Dey et al (2010); Jamróz et al (2017); Singh et al (2012).

Films as described herein may also be produced by any method known to a person skilled in the art, and for example, by the methods described in Nagaraju et al. (2013), Amin et al. (2015) and Irfan et al. (2016) which include casting and drying (solvent casting or semi-solid casting), extrusion (hot-melt extrusion or solid dispersion extrusion), rolling method, spray technique.

In one example, producing films by hot-melt extrusion comprises: mixing of hydrophilic acid insoluble polymers, addition of minoxidil and plasticizer, extrusion, drying and cutting of the extrusion into films.

In one example, producing films by solvent casting comprises: preparation of a solvent suspension comprising minoxidil, casting of a solvent suspension, drying of the solvent suspension, film stripping, film packaging.

In one example, producing films by solid dispersion extrusion comprises: mixing of minoxidil with a suitable solvent, adding the mixture of minoxidil and solvent to a melted polymer along with immiscible components, cutting of the solid dispersion into a film.

In one example, producing films by the rolling method comprises: preparation of a suspension comprising minoxidil and polymer in water or alcohol, subjecting the suspension to rollers, evaporation of solvent, cutting into film.

Films as described herein may comprise one or more of the following: a film forming agent, a plasticizer, taste modifying agent, surfactant, thickener and/or stabilizer, a saliva stimulating agent, a colouring agent.

"Film forming agent" refers to a polymer capable of forming a film. In an embodiment, the film forming agent may be selected from: hydroxypropylmethyl cellulose (HPMC), hydroxypropyl cellulose (HPC), pullulan, carboxymethyl cellulose (CMC), pectin, starch, polyvinyl acetate (PVA), and sodium alginate.

"Plasticizer" refers to an agent which improves the flexibility and/or decreases the brittleness of a film. In an embodiment, the plasticizer may be selected from: glycerine, sorbitol propylene glycerol, glycerol, caster oil, triacetin, trithyl citrate, acetyl triethyl citrate and other citrate esters.

In an embodiment, the dosage forms and compositions as described herein do not comprise hydroxyapatite.

Any discussion of documents, acts, materials, devices, articles or the like which has been included in the present specification is not to be taken as an admission that any or all of these matters form part of the prior art base or were common general knowledge in the field relevant to the present disclosure as it existed before the priority date of each claim of this application.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

It will be appreciated by persons skilled in the art that numerous variations and/or modifications may be made to the above-described examples, without departing from the broad general scope of the present disclosure. The present examples are, therefore, to be considered in all respects as illustrative and not restrictive.

This application claims priority from Australian Provisional Application No. 2017902722 entitled "Promoting hair growth and treatment of hair loss or excessive hair shedding" filed 12 July 2018.

The steps, features, integers, compositions and/or compounds disclosed herein or indicated in the specification of this application individually or collectively, and any and all combinations of two or more of said steps or features.

### Examples

### Example 1: Preparation of sublingual minoxidil troche

A sublingual minoxidil (0.25 mg) troche was prepared comprising sweetened mango flavour as described below:
*Materials:* Minoxidil, Sweetened Mango Flavour Powder, Base PCCA Rapid Dissolve Tablet Powder^{™}.

### Compounding procedure:

Determine the total weight of minoxidil to be incorporated into each tablet. Subtract the weight from Step 1 from the amount of PCCA Rapid Dissolve Tablet Powder Base. This calculated amount is the weight of PCCA Rapid Dissolve Tablet Powder Base which should be used per each tablet. When using the Rapid Dissolve Tablet Mold, the ingredients required for filling the mold are multiplied by 96. Preheat the convection oven to 110°C. Note: minoxidil is stable at a temperature of 110°C for 30 minutes. Using the principles of geometric dilution, triturate minoxidil, Sweetened Mango Flavor Powder and PCCA Rapid Dissolve Tablet Powder Base together in a mortar and pestle to reduce particle size and obtain a uniform mixture. To fill the Rapid Dissolve Tablet Mold, pour approximately one-half of the formula powder onto the bottom plate of the RDT Mold and begin filling the holes. Add more formula powder to fill all of the holes. Tap the plate on the counter to settle the powder into the holes. Add additional powder to level fill the holes. Remove excess powder between the holes and reserve it. Pressing the RDTs in preparation for baking requires a 3 step process. Step 1, press the powder with the RDT Mold Top Plate. Remove the top plate and fill the holes with a portion of the remaining formula powder. Remove excess powder between the holes and reserve it. Step 2, press again with the top plate. Remove the top plate and fill the holes with the remaining formula powder. Step 3, press again with the top plate and remove it. Bake the filled RDT bottom plate on the middle rack in the convection oven between 100°C and 110°C for 30 minutes. Remove the RDT mold from the convection oven and allow it to cool at room temperature for no more than 5 minutes. Remove the tablets from the RDT mold and allow the RDTs to cool for an additional 15 to 30 minutes. Package the tablets into the Amber Round Blister Pack. The beyond use for troches prepared using the above protocol is estimated to be 180 days.

### Example 2: Preparation of sublingual minoxidil polyglycerol troche

A sublingual minoxidil (0.25 mg) polyglycerol troche was prepared as described below:
*Materials:* Minoxidil, Silica Gel (PPTD) Micronised, Flavoring, Base PCCA Polyglycol Troche^{™}.

*Compounding procedure:* In a beaker, melt PCCA Polyglycol Troche Base was melted at 50°-55°C while stirring (melting was not performed in the microwave). Using a mortar and pestle, minoxidil and Silica Gel are triturated together to a fine powder. The powder from Step 2 was sifted into the melted Base and stirred until evenly dispersed. Next, the flavour was then added and the composition mixed well. The mixture was then poured into mold and allowed to congeal at room temperature. The beyond use for troches prepared using the above protocol is estimated to be 180 days.

### Example 3: Preparation of 0.45mg sublingual minoxidil or placebo mini-tablet

A sublingual minoxidil tablet was prepared as described below:
*Materials:* Minoxidil, Microcrystalline Cellulose (PH-102) (Fargon, #2059/165002784), Polyethylene glycol 1450 MW NF (Base A; PCCA, #30-1013) and Silica Gel PPTD Micronized (PCCA #30-1009).

*Compounding procedure:* Cover one side of the mold with Lab Film-Parafilm M^{™}. In an appropriate size beaker, melt Base A at 50°C. Using a mortar and pestle, triturate minoxidil or placebo with microcrystalline cellulose (PH-102) trituration and silica gel together to a fine powder. Sift the powder into the melted Base while stirring. The use of a strainer helps to ensure small particle size. Discontinue heat and stir until suspended. Using an appropriate size syringe, distribute the melted mixture into Tablet Triturate 200 mg mold and allow to congeal at room temperature. The final product is to be protected from sunlight and stored in an air-tight container.

### Example 4: Preparation of 1mg minoxidil capsules (comparative example)

An oral minoxidil tablet was prepared as described below:
Materials: Minoxidil, Microcrystalline Cellulose (PH-102) (Fargon, #2059/165002784).

Compounding procedure: Using the Principles of Geometric Dilution, mix Minoxidil and Microcrystalline Cellulose (PH-102) together with trituration in a mortar and pestle. Encapsulate in size #3 capsules, using Profiller 1000 capsule machine. The beyond use date after compounding is estimated to be 180 days.

### Example 5: Assessment of sublingual minoxidil on patient blood pressure

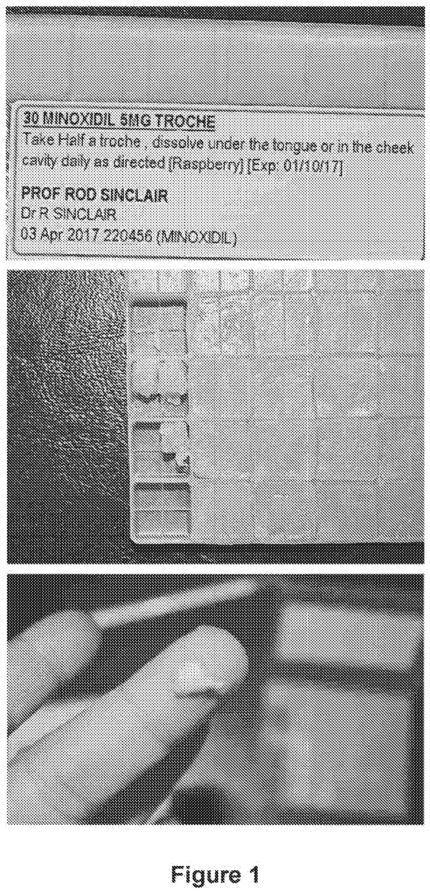

Individuals (5) were dosed with sublingual minoxidil (2.5 mg formulated as a sublingual troche as shown in Figure 1) and blood pressure was monitored for 6 hours post administration. No adverse symptoms, postural hypotension or change in blood pressure was noted in the time period assessed.

### Example 6: Assessment of sublingual minoxidil on hair numbers

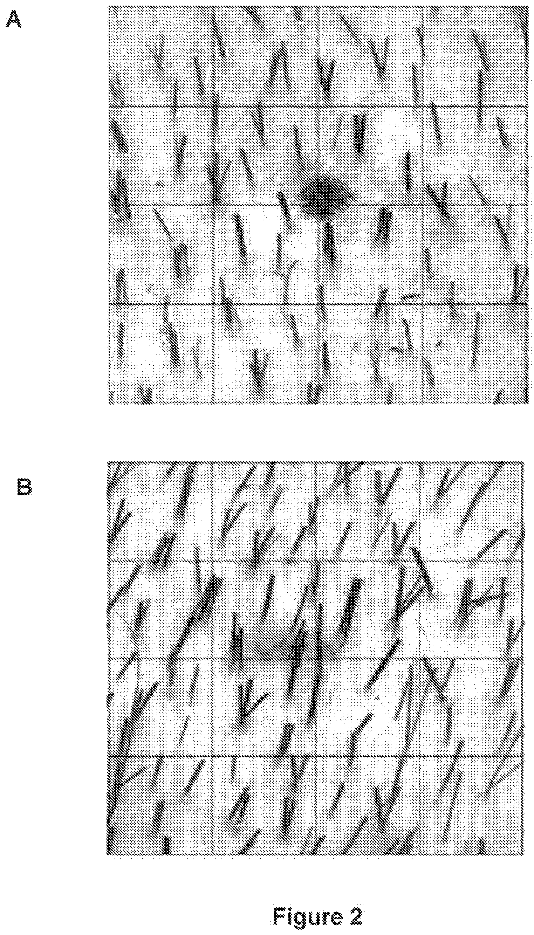

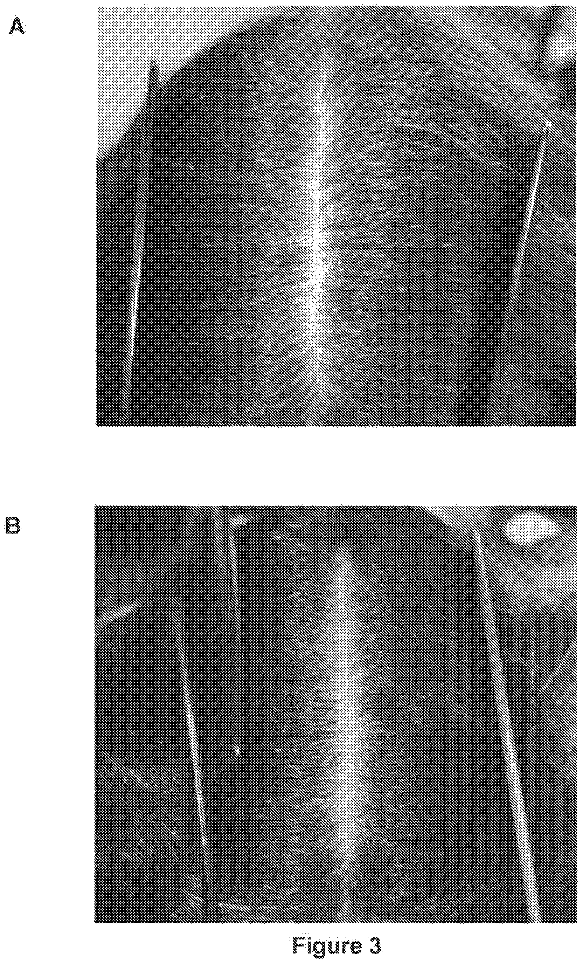

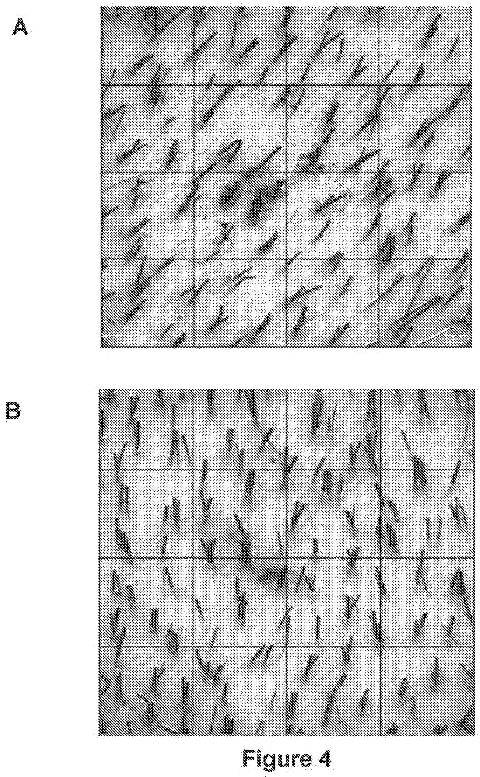

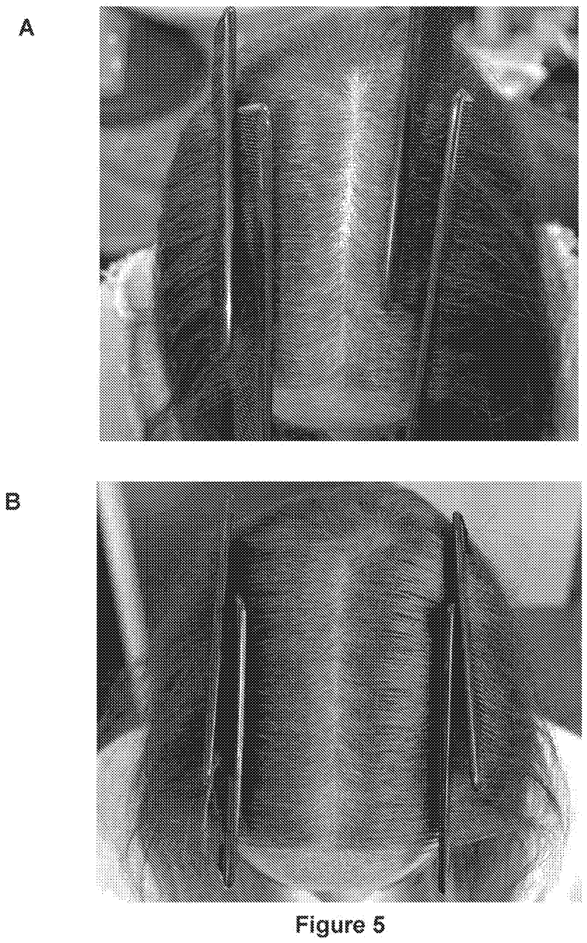

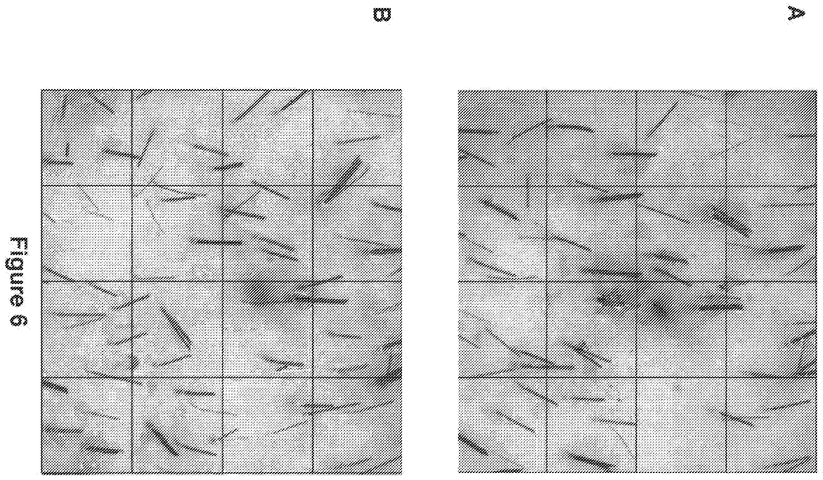

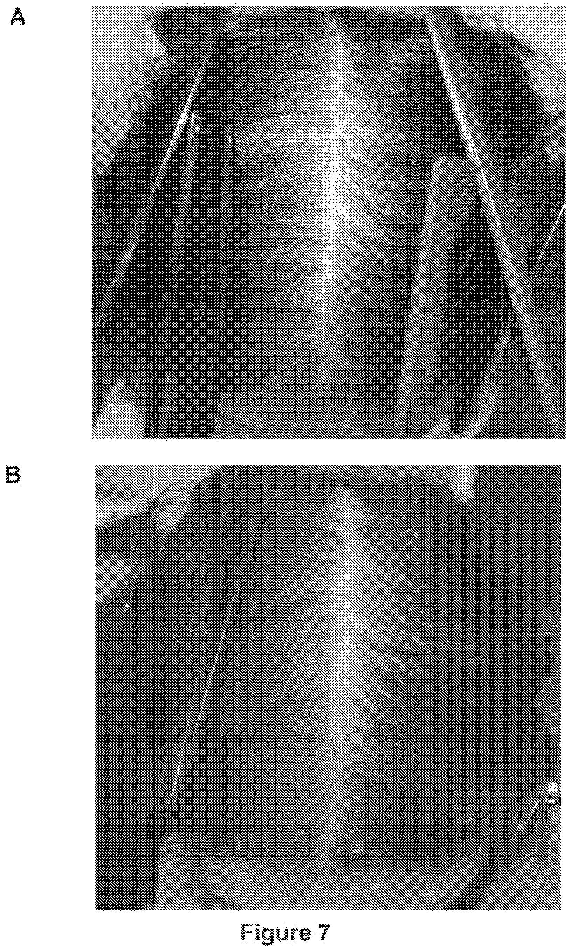

Three women were treated with sublingual minoxidil (0.25 mg formulated as a sublingual troche as prepared in Example 2) daily for 12 weeks. As shown in Figures 2 to 7 and Table 1 an increase in total hair count was observed in all three patients. The increase ranged from a 19.2% increase to 37.7%.

**Table 1: Hair count at base line and 12 weeks post sublingual minoxidil treatment.**

| **Patient** | **Sinclair Scale** | **Baseline Terminal hair Count** | **Hair Count at week 12** | **Percentage increase** |
|---|---|---|---|---|
| 1 | 3 | 100 | 123 | 23% |
| 2 | 2 | 151 | 186 | 23.2% |
| 3 | 4 | 50 | 71 | 42% |

### Example 7: Pharmacokinetics of Sublingual Minoxidil for the Treatment of Male Pattern Hair Loss

### Aim:

To investigate the pharmacodynamics and hypotensive effect of sublingual dosing of minoxidil in men and women with AGA.

### Summary:

Men with a Hamilton Norwood stages III vertex, IV or V and women with Sinclair stages 2-5 pattern hair loss were treated with minoxidil 0.45 mg sublingually. Hair counts were performed on dermatoscopic images taken at baseline and after 6 weeks. Serum levels of minoxidil and blood pressure were assessed at baseline, immediately after first dose, and again at 15 minutes, 30 minutes, 1, 2, 4, 6 and 24 hours. Steady state serum levels were measured after 8 weeks of daily dosing. 13 patients had hair counts performed. At 6 weeks all patients had increased terminal hairs on both frontal and vertex areas. The mean increase in frontal scalp terminal hairs was 69.13 hairs/cm² (range 4-133 hairs/cm²) and vertex scalp terminal hairs was 97.38 (range 31-253 hairs/cm²). All patients showed either mild or moderate improvement in scalp global photographs at 6 weeks serum levels were measured in 5 patients after their first dose of minoxidil. The peak serum levels all occurred within 1 hour an there was no detectable minoxidil in the serum after 2 hours. The mean peak level was 1.42 ng/ml (range 2.6- 0.6 ng/ml). Minoxidil 0.45 sublingual Blood pressure was unaffected. No adverse events were noted. Among patients who had received minoxidil 0.45 mg sublingually daily for a minimum of 6 weeks no minoxidil was detected in serum samples. Mean peak serum levels following a single sublingual dose of minoxidil 0.45mg occurred at 1 hour and was 1.42 ng/ml, which is less than the mean peak serum levels seen after topical application of 5% minoxidil lotion (1.8ng/ml) and well below the minimum dose threshold for any hemodynamic effect (20 ng/ml). Minoxidil is undetectable in the after 2 hours and does not accumulate with continuous dosing. Hair regrowth seen with once daily sublingual dosing of minoxidil 0.45 mg is greater and detected earlier than with topical or oral minoxidil. We propose that sublingual minoxidil has a superior safety and efficacy profile than either topical or oral minoxidil.

### Introduction:

Minoxidil is a piperidinopyrimidine derivative and a potent vasodilator that is effective orally for severe hypertension. With respect to its hemodynamic effects, minoxidil is a pro-drug, activated by the hepatic enzyme sulfotransferase into minoxidil sulfate. Oral minoxidil in doses of 5 to 100mg daily can lead to a profound reduction in blood pressure in hypertensive patients but had minimal effect on the blood pressure of normotensive individuals. Reflex tachycardia and sodium retention can occur as a consequence of increased sympathetic activity. Twelve hour steady state intravenous infusions of minoxidil were used to investigate the serum concentration/hemodynamic response relationship. The serum concentration threshold for any hemodynamic response is 20 ng/ml (Ferry et al., 1996).

In humans, oral minoxidil is well absorbed through the gastrointestinal tract (>90%). At one hour post- dose, oral doses of 2.5mg and 5mg minoxidil provided the peak serum concentrations of 18.5 and 41ng/mL, respectively. Oral minoxidil was approved by the FDA for the treatment of hypertension in 1979. It was first noticed to improve hair loss in male androgenetic alopecia in 1980. When applied topically, minoxidil has been shown to arrest hair loss or to induce mild to moderate hair regrowth in approximately 60% of men with MPHL. Topical minoxidil, at 5% concentration, was shown to increase hair count 12.3% on average at 12 months. Topical minoxidil was first approved by the FDA in 1988 for the treatment of male pattern hair loss. It appears to be a safe therapy with side effects only of local irritation and a low incidence of contact dermatitis. If treatment is stopped, clinical regression occurs within 6 months, to the state of baldness that would have existed if treatment had not been applied. For patients to maintain any beneficial effect, applications must continue indefinitely.

Topical minoxidil has minimal effect on blood pressure or pulse rate. The effect of topical Minoxidil on hair growth is dose related. Approximately 1.5-4% of minoxidil is absorbed topically. Application more frequently than twice daily does not further increase absorption as the initial dose saturates the skin for a period of time longer than the dosing intervals examined.

Mean serum concentrations of minoxidil after topical application to the scalp twice daily is 0.7 ng/ml, with 2% minoxidil (20mg/ml) and 1.8ng/ml with 5% minoxidil (50mg/ml). Individual variations in cutaneous absorption lead to considerable differences in the peak levels and approximately 10% of patients using minoxidil 5% lotion achieve levels > 10ng/ml. The highest serum levels recorded was 18.2 ng/ml which is below the serum level (20 ng/ml) where haemodynamic effects have been first reported.

In 2006, 5% minoxidil foam was also approved for male AGA and in 2014 it was approved for female pattern hair loss. Increased cutaneous absorption occurs with the foam and mean serum levels of 11.5 ng/ml is seen when 3 grams of foam is applied topically to the scalp.

Oral minoxidil (a dose that is swallowed and absorbed intestinally) has also been used to treat androgenetic alopecia. In a head to head study comparing 24 weeks of treatment of topical 5% minoxidil once daily to oral minoxidil 1 mg daily (data on file), 41 women aged 20-68 with Sinclair stages 2-5 FPHL were treated. The mean baseline hair density in both groups was 95 hairs/cm². The mean increase in terminal hair count in the topical minoxidil group was 7 /cm², and 27/cm² in the oral minoxidil group. As the hair bearing area of an average scalp is 630cm², the number of scalp hairs at baseline is approximately 60,000. A 7 hair/cm² increase in hair density would produce 4600 additional hairs while a 27 hair /cm² increase in density would lead to 17,000 additional hairs.

In order to investigate the pharmacodynamics and hypotensive effect of sublingual dosing of minoxidil the following was assessed:
1. Investigated the effect of sublingual minoxidil 0.45 mg (as prepared in Example 3) once daily on hair regrowth in 26 men with androgenetic alopecia and 10 females.
2. Measured minoxidil serum levels in 7 patients after a single dose of minoxidil (as prepared in Example 3). Serum levels of minoxidil and blood pressure were assessed at baseline, immediately after first dose, and again at 15 minutes, 30 minutes, 1, 2, 4, 6 and 24 hours
3. Measured steady state minoxidil serum levels in patients who have been taking sublingual minoxidil 0.45 mg (as prepared in Example 3) once daily for a minimum of 6 weeks and compared levels to patients who have been ingesting minoxidil capsules in various doses for a minimum of 6 weeks.

### Results:

*1. Hair count* - *global photography:* Blinded assessment outcome indicates that 5 of the patients receiving 0.45mg sublingually showed a slight increase in midline hair density 8 weeks following commencement of study medication. Clinic assessment of 2 patients on minoxidil 0.45 mg sublingually daily for a minimum of 6 weeks showed a moderate increase (+2) in hair density.
*1. Hair count* - *macrophotography:* Hair counts were performed on 13 patients on minoxidil 0.45 mg sublingually daily for a minimum of 6 weeks. All patients presented with an increase in hair counts on both frontal and vertex areas. Baseline mean hair counts in frontal scalp terminal hairs were 189.33 hairs/cm² (range 110-249 hairs/cm²) and vertex scalp terminal hairs were 185.25 (range 64-313 hairs/cm²). The mean increase in frontal scalp terminal hairs was 69.13 hairs/cm² (range 4-133 hairs/cm²) and vertex scalp terminal hairs was 97.38 (range 31-253 hairs/cm²). Hair counts for frontal scalp shows a fold increase minimum of 1.02 and a max of 1.9 (range 110-358 hairs/cm²). Scalp vertex area fold increase minimum is 1.13 and maximum is 5-fold (range 64-317 hairs/cm²).
2. *Minoxidil plasma levels after single dose:* 5 patients received sublingual minoxidil and 7 patients received placebo. The peak serum levels all occurred within 1 hour and there was no detectable minoxidil in the serum after 2 hours. The mean peak level was 1.42 ng/ml (range 2.6- 0.6 ng/ml). This is 10 times lower than the peak level seen with the 2.5 mg minoxidil tablet and 25 times lower than the peak dose seen with the 5 mg minoxidil tablet. At the time of peak concentration of minoxidil there was no change in blood pressure detectable in any patient.
3. *Minoxidil steady state levels:* 2 patients who had received minoxidil 0.45 mg sublingually daily for a minimum of 6 weeks were assayed. No minoxidil was detected in any of these patients. 30 patients received sublingual minoxidil for between 2 and 12 months. No side effects were reported. No biochemical abnormality was detected. No patient developed hypertrichosis, tachycardia, fluid retention or postural hypotension and there was no reduction in the mean blood pressure. All participants completed 24 weeks of treatment.

Peak serum levels following a single sublingual dose of minoxidil 0.45mg occur at 1 hour and minoxidil is undetectable in the serum after 2 hours. The mean peak serum level was 1.42 ng/ml, which is more than the mean peak serum level seen after topical application of minoxidil 2% (0.7ng/ml) but less than the mean peak serum levels seen after topical application of 5% minoxidil lotion (1.8ng/ml) and well below the minimum dose threshold for any hemodynamic effect (20 ng/ml). Minoxidil does not accumulate with continuous sublingual dosing and minoxidil was not detected in the serum of any of patients who had been using sublingual minoxidil.

Hair regrowth seen with once daily sublingual dosing of minoxidil 0.45 mg is greater and detected earlier than with topical or oral minoxidil. The mean increase in frontal scalp terminal hairs was 69.13 hairs/cm² (range 4-133 hairs/cm²) and vertex scalp terminal hairs was 97.38 (range 31-253 hairs/cm²). This represents a 36.5% increase from baseline in hair count over the frontal scalp and a 52.5% increase from baseline on the vertex scalp. Historical data suggests that minoxidil lotion produce a 7 hair/cm² increase in hair density and oral minoxidil a 27 hair/cm² increase in hair density.

We propose that sublingual minoxidil has a superior safety and efficacy profile than either topical or oral minoxidil. Minoxidil when ingested orally is absorbed through the gastrointestinal mucosa and transported to the liver where it undergoes extensive first pass metabolism. The metabolites are either inactive or in the case of minoxidil sulfate, active systemically but unlikely to diffuse into the skin and reach the target tissue - the hair follicle bulb.

When minoxidil is ingested sublingually it is absorbed through the oral mucosa into the circulation and delivered directly to the hair follicle. Intra-follicular conversion to the active metabolite minoxidil sulfate by the enzyme sulfotransferase (found in the hair bulb outer root sheath epithelium) traps the minoxidil sulfate within the hair follicle as the molecule is too large to diffuse out.

Circulating minoxidil does not have any haematological effect as it is a pro-drug. Circulating minoxidil is rapidly excreted and undetectable in the circulation after 1 hour.

Cutaneous absorption of topical minoxidil varies fourfold from 1-4%. Consequently some patients experience systemic symptom following topical application of minoxidil lotion. As the sublingual absorption of minoxidil is nearly 100%, there is minimal variation from person to person in peak serum levels and so systemic symptoms were not seen in any of our patients and would be much less likely to occur than with topical minoxidil. As person to person variation in hepatic sulfotransferase is well recognized, systemic symptoms may be seen in some patients following oral ingestion of minoxidil, even when used in low doses. As sublingual dosing bypasses hepatic first pass metabolism, systemic effects are less likely to occur than with oral minoxidil.

Surprisingly sublingual minoxidil 0.45 mg grows more hair than oral minoxidil 1 mg and most likely reflects greater hair follicle bio-availability.

### Example 8: Assessment of low-dose sublingual minoxidil in patients diagnosed with either female (FPHL) or male pattern hair loss (MPHL)

### Aim:

To investigate the pharmacodynamics and hypotensive effect of sublingual dosing of minoxidil in men and women diagnosed with either female (FPHL) or male pattern hair loss (MPHL).

### Introduction:

Both FPHL and MPHL are produced by androgenetic alopecia (AGA). AGA is the most common cause of hair loss in the community. In AGA, hair loss is produced by androgen mediated hair follicle miniaturization in genetically susceptible individuals. The morbidity is predominantly psychological, however early-onset MPHL is associated with increased risk of prostate cancer and cardiovascular disease and can cause reduced physical attractiveness, anxiety and occasionally depression. In women, FPHL is associated with hypertension, hypercholesterolemia, late-onset diabetes and may be a feature of polycystic ovary syndrome. FPHL can also cause reduced self-confidence, physical attractiveness, anxiety, mood and depression.

Minoxidil lotion has been shown to be effective in converting vellus hair to terminal hair in 30% of patients when applied in a 2% solution with a 10% propylene glycol water base. 1 ml of the solution is applied to the scalp twice daily. A temporary telogen effluvium may occur within the first 8 weeks. Treatment with a 5% solution yields superior hair growth compared to the 2% solution in both males and females. Foam formulations without propylene glycol cause fewer cutaneous side effects.

Published clinical studies in MPHL are limited to topical minoxidil treatment. Histologically, patients who have had treatment with topical minoxidil have an increase in the number of hair follicles in anagen and have a thicker hair diameter.

### Study Design:

This study is a single-centre, randomized, double-blind study comparing the effects of sublingual minoxidil to placebo. Participants receive study medication for 24 weeks followed by a follow-up visit 4 weeks later. Objectives and endpoints are summarised in Table 2.

The aim is to enrol 40 participants. Male and female participants will be randomized to receive either 1) 0.45 mg sublingual minoxidil once daily or 2) placebo.

Study visits will be scheduled at Screening and on Study Weeks 0, 8, 16, 24 and 28. Study Week 2 (Visit 3) will be a phone call consult with Site staff.

**Table 2: Study objectives and endpoints.**

| **Primary Objective:** | **Primary endpoints:** |
|---|---|
| To evaluate the efficacy of low dose oral minoxidil compared to placebo on hair density in patients with FPHL or MPHL. | 1) Change from Baseline of quantified non-vellus hair counts to Week 24. |
| | 2) Assess the subjective impact of hair growth and quality by participant's completion of the Sinclair Scale, Women's AGA Quality of Life Questionnaire (WAA·QOL) and the hair shedding scale for female patients. Dermatology Life Quality Index (modified) and Men's Hair Growth Questionnaire (MHGQ} for male patients. |

| **Secondary Objectives:** | **Secondary endpoints:** |
|---|---|
| 1) Assess the investigator assessment scale against hair density by global photography. | 1) Assess change in hair growth through global photographs of the vertex and mid-frontal scalp assessed by a panel of 3 independent hair experts using the Investigator Assessment scale. |
| 2) Assess the subjective impact of oral minoxidil on participant's subjective improvement in hair growth and quality | 2) Assess the subjective impact of oral minoxidil compared to placebo on participant's subjective improvement in hair growth and quality using the Kingsley Alopecia Profile (KAP). |

| **Safety Objective:** | **Safety endpoints:** |
|---|---|
| Characterize the safety and tolerability of oral minoxidil in participants. | 1) Incidence of treatment-emergent adverse events (AEs). |
| | 2) To assess the safety and tolerability of low-dose oral minoxidil in participants, which will be determined by electrocardiogram (ECG) abnormalities and presence of fluid retention, palpitations, rash, hypertrichosis, nausea and vomiting. |

### Participants:

Males and females aged between 18 years and 65 years clinically diagnosed with MPHL and FPHL respectively. Patients will be in good health and have met/ will meet the inclusion and exclusion criteria outlined below.

Sublingual minoxidil has been/will be provided as a single 0.45 mg dose of active minoxidil. Each participant will be randomly assigned to a group for the duration of the study at treatment visit 1.

Inclusion criteria: Males and females between 18 and 75 years of age, inclusive; clinical diagnosis of MPHL with Norwood-Hamilton Classification scores of 3(111) Vertex, 4(IV), 4(IV)a, 5(V), 5(V)a and 6 or FPHL with the Sinclair scale scores of 2 to 5; female patients of childbearing potential agree to use an adequate method of birth control and have a negative urine pregnancy test at Screening Visit and all subsequent visits; in good general health, as determined by the Investigator; willing and able to attend all study visits and comply with treatment plan and required laboratory testing; willing to maintain the same hairstyle as at the Screening Visit for the duration of the study; for patients who dye their hair to conceal canites, be willing to attend for hair colour 2-3 days prior to the treatment visit; willing to use a mild non-medicated shampoo and conditioner for the duration of the study; willing to receive a 1 millimeter temporary scalp tattoo; able to comprehend and willing to sign and date an informed consent form (ICF).

Exclusion criteria: treatment for PHL including 5 alpha reductase anatagonist medications (e.g., finasteride, dutasteride), anti-androgenic therapies (e.g., spironolactone, flutamide, bicalutamide, cyproterone acetate), topical or oral minoxidil during the study or within 12 weeks prior to treatment visit 1; use of any scalp hair growth products (ketaconazole shampoo, topical prostaglandin or prostanoid treatment, aminexil, nioxin, Fusion Hair 101, platelet rich plasma injections, low level LED light treatment) during the study or within the 6 weeks prior to treatment visit 1; scalp hair loss on the treatment area, due to disease, injury, or medical therapy or other types of hair loss that could confound clinical or photographic assessment of response; skin disease (e.g., psoriasis, atopic dermatitis, skin cancer, eczema, sun damage, seborrhoeic dermatitis), cuts and or abrasions on the scalp or condition (e.g., sunburn, tattoos) on the treatment area that, in the opinion of the Investigator, might put the participant at risk or interfere with the study conduct or evaluations; history of hair restoration surgery; current use of an occlusive wig, hair extensions, or hair weaves that might interfere with assessment of response; history of hypersensitivity or allergies to any ingredients contained in the study medication; known allergy to dye pigments; current participation in any other investigational drug or medical device trial, which included administration of an investigational study medication or medical device, or within 3 months or 5 half-lives of the investigational product, whichever is longer, prior to the Screening Visit; known sensitivity to the investigational product; participants with a history of clinically significant cardiac arrhythmia as determined by the Investigator; participants with clinically significant findings from medical history, clinical laboratory tests, electrocardiogram (ECG), or vital signs that, in the opinion of the Investigator, could interfere with the objectives of the study or put the participant at risk; use of antihypertensive medication (cardiac & renal co-morbidities); pregnant, planning a pregnancy or nursing a child

36 participants were screened. No. of males/ No. of females screened: 26/10. 7 pulled out after screening (prior to receiving study medication): 2 females, 5 males. 7 screen failed: 3 females, 4 males. General reasons: did not meet minimum criteria for hair loss. Number of participants enrolled (started study medication): 21 (4 females, 17 males). 1 male participant pulled out after receiving medication (advised site of headaches after 4 weeks).

**Table 3. Overall disposition of patients and analysis populations**

| **Analysis population** | **n (%)** |
|---|---|
| Participants screened for the study | 36 (90%) |
| Enrolled study population (enrolled) | 21 (52.5%) |
| Screen failed population | 7 (19.4%) |
| Week 8 study population | 12 (57.4%) |

*0.45 mg sublingual minoxidil dose:*
The sublingual minoxidil dose was prepared as described in Example 3.

### Subgroups of data analysed:

Number of participants completed Week 8: 12 (2 females, 10 males). As the study is ongoing, the data presented are participants who have received (0.45mg sublingual minoxidil) for 8 weeks. This timepoint represents a third of the planned treatment period for the study (24 weeks). Data from these 12 participants have been analysed at the 8 week mark. Analysis was performed blind as the study is still ongoing.

### Data groups for this study:

Demographics; investigator scalp assessment: Sinclair Scale (Female) or Norwood-Hamilton (Male) classification grading; Global Photography; Macrophotography; History of hairloss; Sublingual minoxidil pharmacokinetics; Investigator scale assessment at Week 8 (Norwood-Hamilton scale for males and Sinclair Scale for females); Patient reported outcomes; Dermatology Life Quality Index (males); Men's Hair Growth Questionnaire (males); Women's AGA Quality of Life Questionnaire (females); Sinclair Scale (females) and Hair Shedding Scale (all).

### Clinical assessments:

Non-fasting blood and urine samples for clinical laboratory analysis will be collected by a qualified staff member at screening and weeks 8, 24 and 28. All samples will be processed and analysed off-site by a commercial pathology laboratory. At a minimum, the following tests will be conducted:

Chemistry panel: Albumin, alkaline phosphatase, alanine transaminase, aspartate transaminase, blood urea nitrogen, bicarbonate, calcium (corrected total), chloride, urea, creatinine, glucose, lactate dehydrogenase, potassium, sodium, bilirubin (total).

Haematology: Haemoglobin, hematocrit, erythrocytes (RBC), leukocytes (WBC) with differential and platelet count.

Urinalysis: Colour, clarity, specific gravity, pH, protein, glucose, ketones, nitrite, leukocytes, and occult blood. Urine microscopy will be performed if urinalysis values are out of range and the investigator deems that microscopy is clinically warranted.

The Investigator or medical designee must review the results of each participant's Screening Visit clinical laboratory test prior to the first treatment visit. The participant must not be randomized on Treatment Visit Day 1 if any of the Screening Visit results are outside normal range for the laboratory and, if in the opinion of the Investigator, are clinically relevant. The results of the clinical laboratory tests will be reported on the laboratory's standard reports.

### Medical history, vital signs and electrocardiogram results are also collected.

### Demographics/vital signs/safety blood pressure:

Of the 21 participants enrolled in the study, 12 had completed their Week 8 visits. These 12 participants are referred to as the study population from here on. A significant percentage of participants who enrolled identify as Caucasian (8 participants, 66.6%), 3 identify as Asian (25%) and 1 African American (8%). The mean age of participants is 48.25 with the youngest participant screened at age 25 and oldest at 61 years of age. Both are male participants. No comorbidities were reported at screening, however hypolipidemia was reported for several patients at screening and was considered as a medical history entry. The mean weight for the two female participants was 68.8kg. Of these two, one participant was categorized as obese based on BMI calculation (34.02). The average weight for male participants was 89.4kg. The heaviest male weighs 135kg with a BMI of 39.4 placing him in the obese category. No significant adverse events have been reported at all participants Week 8 visit. Safety blood profile returned readings within the normal range. Blood pressure readings taken at day of randomization and subsequent to Week 8 was not significant. Respiratory rate and tympanic temperature were also within normal range and was also not significant for all participants. Demographics of the 12 participants in the study population are described below in Table 4.

**Table 4. Demographic and baseline characteristics - Enrolled population**

| **Parameter** | **Statistics** | **Visit 1 (baseline)** | | | | | |
|---|---|---|---|---|---|---|---|
| Age [years] | | | | | | | N=12 |
| | Mean (SD) | | | 48.25 (8.26) | | | |
| | Median (range) | | | | | 50 (25 - 61) | |
| Sex | | | | | | | N=12 |
| Female | n (%) | | | | 2 (16.67) | | |
| Male | n (%) | | | | 10 (83.33) | | |
| Weight [kg] | | | | | | | N=12 |
| | Mean (SD) | | 85.98 (18.68) | | | | |
| | Median (range) | | | | 80.95 (59 - 135) | | |
| BMI [kg/m²] | | | | | | | N=12 |
| | Mean (SD) | | | 28.72 (4.31) | | | |
| | Median (range) | 27.21 (24.04 - 39.44) | | | | | |
| Systolic BP [mmHg] | | | | | | | N=12 |
| | Mean (SD) | | 126.75 (19.87) | | | | |
| | Median (range) | | | | 127.5 (99 - 174) | | |
| Diastolic BP [mmHg] | | | | | | | N=12 |
| | Mean (SD) | | | 82.3 (14.94) | | | |
| | Median (range) | | | | 77.5 (60 - 114) | | |

| | | | | | | | |
|---|---|---|---|---|---|---|---|
| *SD* = *Standard deviation* | | | | | | | |

### Investigator scalp assessment: Sinclair scale (female) or Norwood-Hamilton (Male) classification grading

The minimum criteria for hair loss severity in female participants is a Grade 2 and maximum is Grade 5. ISA at Screening was Sinclair Scale 2 and 3 for each female participant. At the Week 8 visit slight increase from grade 3 to 2 was observed by the Investigator for one of the female participants. However, no change was observed for the other. As the study is ongoing and medication blind still stands, the final outcome of change in hair density (at Week 24) cannot be concluded in this report. The minimum criteria for enrolment for male participants is Grade III-Vertex and maximum is VI. Two of the male participants analysed in this data group met the minimum criteria with the most severe of the 10 male participants being a grade Va.

### Global photography:

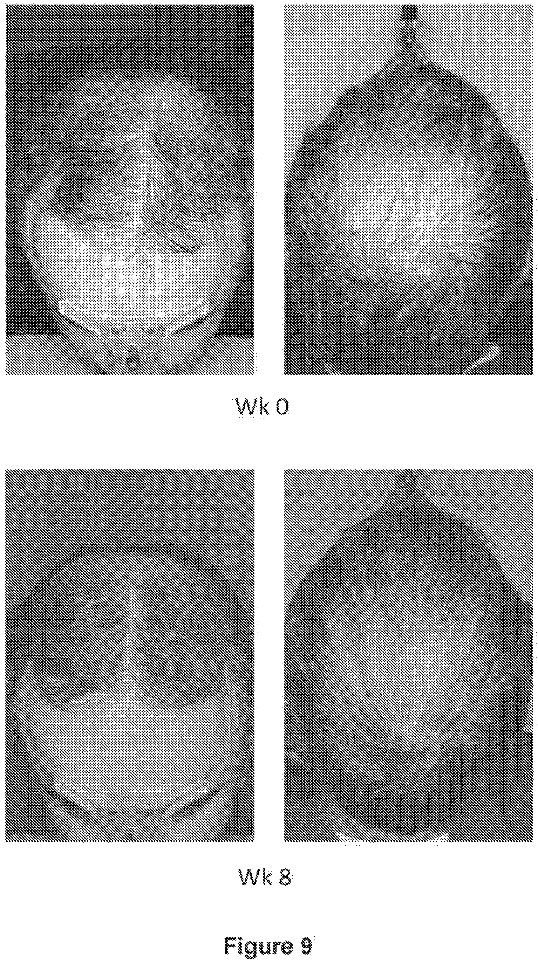

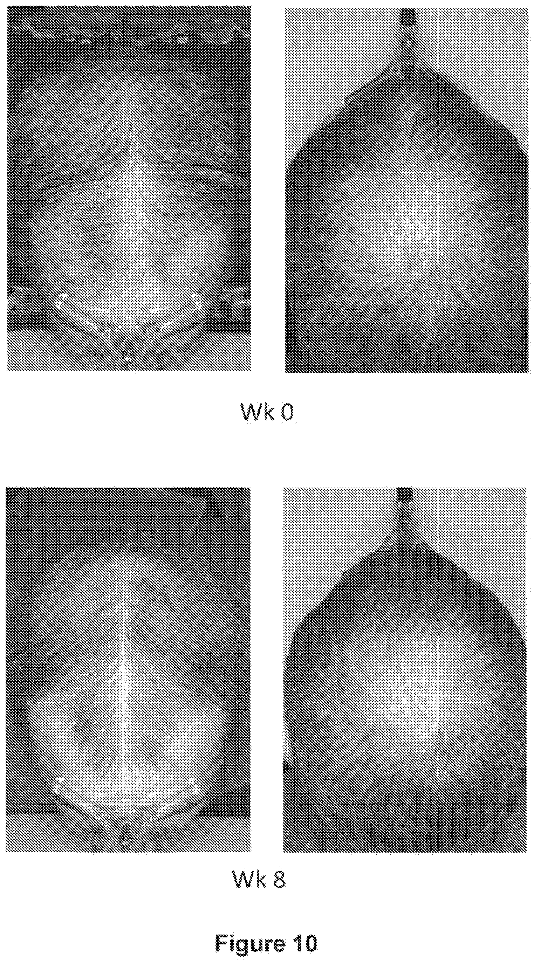

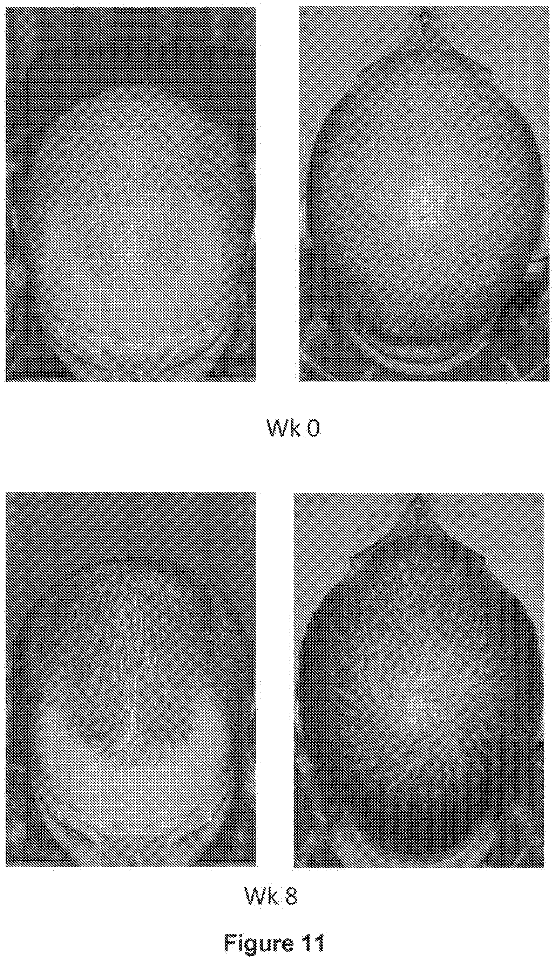

Standardized global photography are taken at Study Weeks 0 (prior to dispensing of medication) and 8. Subsequent Week 16, 24 and 28 visits will also include global photography. A vertex and frontal scalp photograph is taken from a fixed height with a dedicated SLR camera. Current collated global photographs for all 12 patients were assessed by a blinded assessor. Comparison photographs between Baseline and Week 8 was provided to the designated blinded assessor. Assessment outcome indicates that 2 of the 12 participants' photography suggest a decrease in hair density. Eight participants' assessment outcome suggest a slight to moderate increase in hair density. Representative images are shown in Figures 9, 10 and 11.

### Macrophotography:

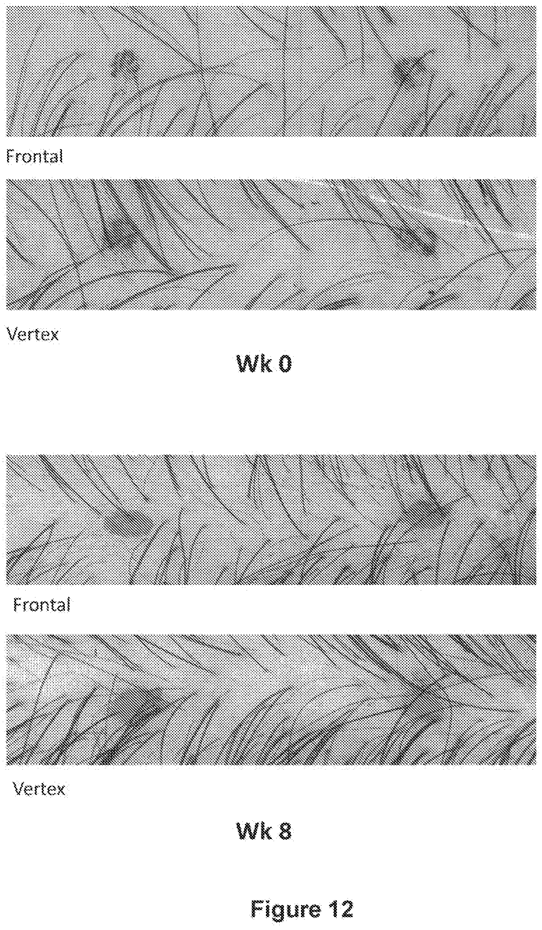

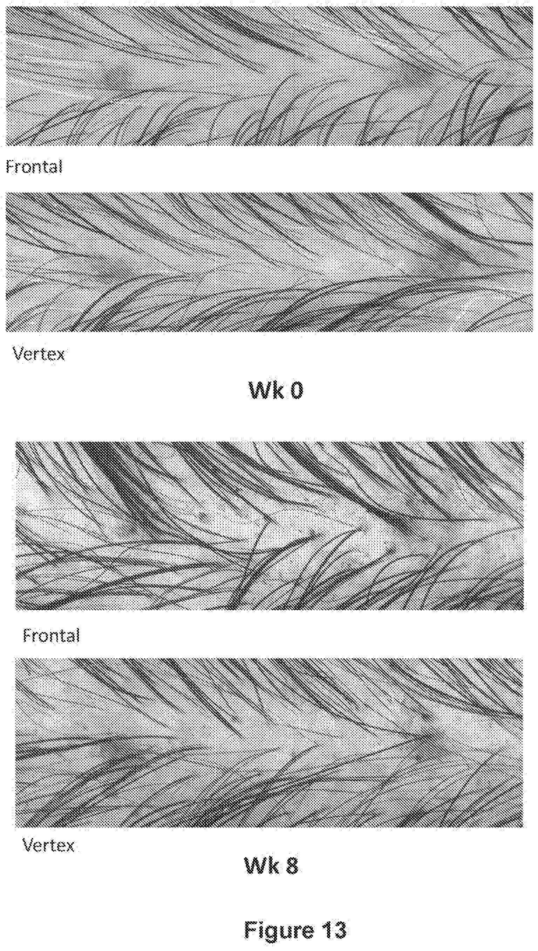

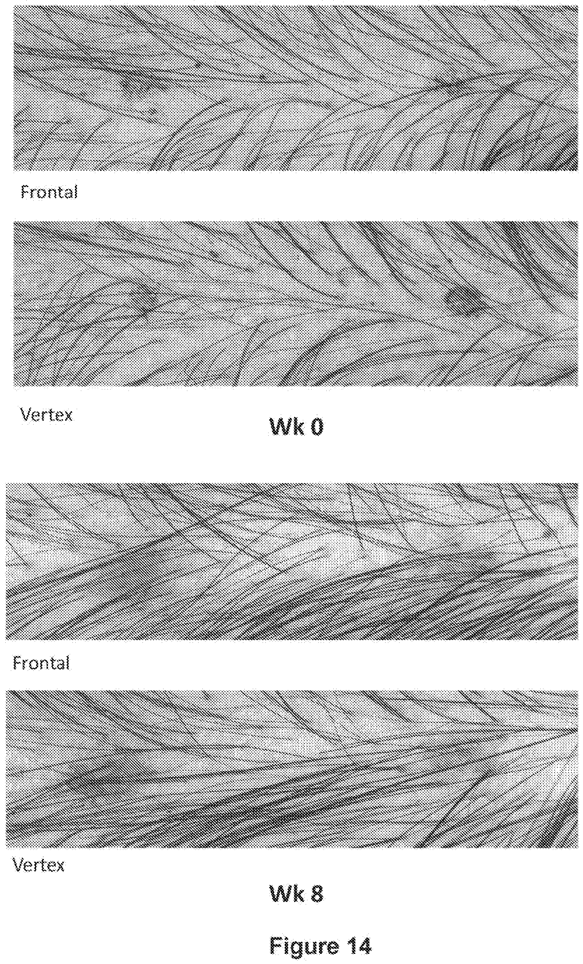

*Standardized scalp macrophotography (SSM):* Standardized scalp colour macrophotographs of the treatment area are collected at Study Weeks 0 (prior to dispensing of medication), 8, 16, 24 and 28. Assessments will be carried out on the Tricholab (TL) Snap acquisition software using the FotoFinder Leviacam. The Leviacam comes with a detachable lens. SSM will be carried out in 2 parts: 1) Hair to hair (H2H)-matched phototrichogram and 2) H2H-matched trichoscopy. The first SSM procedure is carried out at baseline (week 0). A proprietary spot template will be used to assist in determining the points to be marked on the scalp for macrophotography. Pairs of spots (x3) are marked on the patient's scalp: 1 pair for phototrichogram and 2 for phototrichoscopy spots. Representative images are shown in Figures 12, 13, 14 and 15.

*Background on H2H-matching:* H2H matching allows identification of individual hair throughout the treatment period, as well as the new hair that appear over the same time (from empty follicles). This will provide a precise number to correlate the effectiveness of the study medication-induced changes. H2H matching provides evaluation of:
1. Hair count & densities, hair follicle positions
2. Hair shaft thickness and its distribution
3. Derivative measures of percentage of vellus & terminal hair, cumulative hair thickness, percentage of follicular units' density, percentage of single hair follicular units, percentage of multiple hair follicular units.

For each of the 3 above parameters, a comparison can be derived from the counts and diameters. H2H-matched procedure can reliably detect very subtle medication effects on shaft diameters of the same hair, which would not be visible on the level of overall averages. The comparative analysis of patient examinations carried out before and after treatment is also performed on the level of individual hair to obtain the most statistically significant of the results (level 2 H2H matching) and gain in depth understanding of the scalp response to medication.

*H2H-matched phototrichogram:* Region of interest (ROI) is defined as the area around the previously marked spot(s). A circular shaving template (diameter of 2cm) is used to shave the ROI and a small cosmetic tattoo is placed on the area for future identification at subsequent visits. Subsequently 3 images are captured of the area, with brushing after each successful image, to allow movement and high accuracy of identifying all individual clipped hair strands. In addition, this procedure allows evaluation of hair growth rate as well as Anagen/Telogen rate.

*H2H-matched phototrichoscopy:* H2H phototrichoscopy allows macrophotography of the hair and scalp ROI without the traditional requirement of shaving or clipping the participant's hair. As previously mentioned, 2 pairs of spots (Frontal and Frontal-vertex areas) are marked on the scalp using the proprietary spot template. Subsequently 3 images are captured of the area, with rebrushing after each successful image, to allow movement and high accuracy of identifying all individual hair strands. Virtual tattoo technology is used to ensure pre-marked spots are aligned and images are identically orientated. Images are all uploaded onto a central server for analysis and comparison reports (between Baseline and subsequent visits).

### History of hair loss:

Eight participants reported maternal or paternal relatives affected with hair loss. Of these eight, 5 reported hair loss on paternal relatives. There are also reports of siblings suffering from hair loss (5 participants), it is unclear if they are male or female siblings. Three of 12 participants reported no family history of hair loss. Less than half of the participants have reported attempts at previous treatments for hair loss. Treatments were primarily with topical minoxidil (Regaine). Duration of treatment of topical minoxidil was not captured nor was the lapse in time between topical minoxidil and commencement of study medication. However, investigators enrolled participants with the relevant criteria noted (Exclusion criteria 1. Treatment for PHL including: 5-alpha reductase antagonist medications (e.g., finasteride, dutasteride); anti-androgenic therapies (e.g. spironolactone, flutamide, bicalutamide, cyproterone acetate), topical or oral Minoxidil during the study or within 12 weeks prior to treatment visit 1.)

### Pharmacokinetics sublingual minoxidil:

The pharmacokinetics of the sublingual minoxidil dose was assessed to determine the concentration of minoxidil in human plasma samples collected from a clinical trial.

*Materials:* Minoxidil, Micropipettor and pipettes, Plasma from human (Sigma Aldrich, St Louis, MO, #P9523-5 mL), C18 column (Ascentis^{®} Express 53822-U Supelco, Sigma-Aldrich, Castle Hill, New South Wales, Australia), Ethyl acetate (EMSURE^{®}, Merck, #1.09623.2511), Methanol (LiChrosolv^{®} LC-MS grade, Merck), and HPLC vials (Grace Discovery Sciences, Epping, VIC, Australia, #12962).

*Solutions and solution preparation:* Plasma from human (Sigma Aldrich, #P9523-5 mL): Add 5 mL of MilliQ water into the bottle as per manufacturers instructions and mix well to constitute the blank human plasma. Minoxidil: Stock solution (1 mg/mL): Dissolve minoxidil (1 mg) in 1 mL of methanol and store in the fridge. To create working solutions: Add 10 µL of the stock solution to 990 µL of methanol to obtain a solution of 10 µg/mL. Add 20 µL of the 10 µg/mL solution to 980 µL of methanol to obtain a solution of 200 ng/mL. Serial dilutions are made from 200 ng/mL working solution to 2.5, 5, 10, 20 and 50 ng/mL. Mobile phase A (0.1% Formic acid in MilliQ water): Add 1 mL of 0.1% formic acid into 1 L of MilliQ water. Mobile phase B (Methanol): LC-MS hypergrade methanol is used as mobile phase B.

*Method:* Control and clinical human plasma samples were defrosted at room temperature, followed by centrifugation at 1,4000 rpm for 5 min. Next, 20 µL aliquots of the minoxidil working solutions were spiked into 180 µL of blank human plasma to achieve final concentrations of 0.25, 0.5, 1, 2 and 5 ng/mL for the calibration samples in 1.5 mL eppendorf tubes. LC-MS grade ethyl acetate (1000 µL) was then added to the mixture for both clinical and calibration curve samples for liquid-liquid extraction and vortexed immediately for 10 s and then placed in the thermostat/shaker for 15 minutes at room temperature. The vortex step is repeated for 5 s. Next, the samples are centrifuged for 5 minutes at 14,000 rpm and the supernatant (980 µL) is transferred to a clean eppendorf tube. It is then evaporated to dryness under a gentle stream of nitrogen gas (approximately 20-25 minutes). Methanol (100 µL) is then added to the eppendorf tubes with residue and vortexed for 10 s followed by centrifugation for 5 minutes at 14,000 rpm. The contents (approximately 95 µL) are transferred to HPLC vials for analysis of LC-MS (8050-2). The residual samples without methanol will be stored in 4°C for later use. For LC MS/MS analysis, 10 µL of each sample is injected into an Ascentis^{®} Express C18 column (2.7 µm particle size, 2.1 x 50 mm internal diameter) in Shimadzu LC MS 8050-2 system using electrospray ionization in the positive mode by multiple reaction monitoring (m/z 210 to 164). The unknown concentration of minoxidil in clinical samples is determined by comparison to the calibration samples using Shimadzu processing system (LC solution version 1.24 SP1).

### Patient reported outcomes:

Initial assessment of Dermatology Life Quality Index (DLQI) for males and Women's AGA Quality of Life Questionnaire (WAA-QOL): Responses by female participants was generally more negative corresponding to a higher score at both Baseline and Week 8 visits. The impact on Quality of Life was more pronounced in comparison to the male participants. No significant difference in scores for Men's Hair Growth Questionnaire responses between Week 0 and Week 8 were noted. A full score of 38 represented dissatisfaction with current hair growth. The mean score was 25 (SD:2.19) at Week 0 and 24.7 (SD:4.08) at Week 8. The median score from all 10 male participants was 24.5 and 25 respectively. Both female patients reported an average of Grade 3 with the Sinclair Scale (Figure 1 above) at Baseline visit and subsequent Week 8 visit. This was consistent with the Investigator's grade of their hair loss severity.

### References

Adams (1998) Biopharm Drug Dispos 19(8):501-15.
Amin et al (2015) Oral film technology: Challenges and future scope for pharmaceutical industry 3(3): 183-203.
Bittencourt et al (2014) Clin Exp Dermatol 39:868-73.
Dey and Maiti (2010) J Nat Sci Biol Med, 1(1):2-5.
Ferry et al (1996) Abstract. Clinical Pharmacology and Therapeutics 59:166-166.
Gad et al (2008) Pharmaceutical manufacturing handbook: Production and processes. Published by Wiley-Interscience.
Harrison and Sinclair (2002) Clin Exper Dermatol. 27:389-5.
Irfan et al (2016) Saudi Pharmacutical Journal 245:537-546.
Jamróz et al (2017) Int J Pharm. S0378-5173(17)30464-7.
Messenger and Sinclair (2006) Br J Dermatol 155: 926-930.
Messenger et al (2010) Eighth Edition. Blackwell Publishing. Oxford. 63.1-63.100.
Nagaraju et al (2013). Curr Drug Delivery. 10(1):96-108.
Rowe et al (2009) Published by the Pharmaceutical Press and the American Pharmaceuticals Association.
Sayeed et al (2014) Pharamceutical Technology. Volume 38, Issue 11.
Sinclair and De Berker (1997) R Dawber Editors. Blackwell Science. Oxford. Inglaterra 151-238.
Sinclair (2004) International journal of dermatology 43:842-843.
Singh et al (2012) Journal of Drug Delivery 4:407-417.
Whiting (1996) Dermatol Clin 14.4 723-731.
Pfizer (2005) Medical Review application number: NDA 21-812 Minoxidil, Men's Rogaine extra strength 5% Topical foam.
Johnson & Johnson (2014) Product Monograph Submission control number: 1732017 Rogain Topical 2% solution.

## Claims

1. A composition comprising minoxidil for use in a method for the treatment of hair loss or excessive hair shedding in a subject or for promoting hair growth in a subject by administering to a subject an effective dose of minoxidil through the sublingual mucosa.

2. A composition comprising minoxidil for use in a method for the treatment of hair loss or excessive hair shedding in a subject or for promoting hair growth in a subject according to claim 1, wherein the dose of minoxidil is in a form which disintegrates in the presence of saliva.

3. A composition comprising minoxidil for use in a method for the treatment of hair loss or excessive hair shedding in a subject or for promoting hair growth in a subject according to any one of the claims 1 or 2, wherein the dose of minoxidil
a) results in a minoxidil blood concentration of 0.25 ng/mL to 10 ng/mL; and/or
b) is within the range from 0.05 mg to 3 mg, or from 0.1 mg to 2.5 mg, or from 0.15 mg to 2 mg, or from 0.15 mg to 1.5 mg, or from 0.15 mg to 1 mg, or from 0.15 mg to 0.8 mg, or from 0.15 mg to 0.5 mg, or from 0.5 mg to 0.25 mg, or is 0.1 mg, or is 0.15 mg, or is 0.24 mg, or is 0.25 mg, or is 0.45 mg, or is 1.35 mg.

4. A composition comprising minoxidil for use in a method for the treatment of hair loss or excessive hair shedding in a subject or for promoting hair growth in a subject according to any one of the claims 1 to 3 wherein the dose of minoxidil is administered at least every 3 days, at least every 2 days, or at least daily.

5. A composition comprising minoxidil for use in a method for the treatment of hair loss or excessive hair shedding in a subject or for promoting hair growth in a subject according to any one of the claims 1 to 4, wherein the dose of minoxidil is in a form selected from a: strip, wafer, pellet, film, troche, tablet, lipid matrix tablet, capsule, pill, granule, pellet, powder, drop, spray and lozenge.

6. A composition comprising minoxidil for use in a method for the treatment of hair loss or excessive hair shedding in a subject or for promoting hair growth in a subject according to any one of the claims 1 to 5, which further comprises administering a:
aldosterone antagonist, 5α-reductase inhibitor, non-steroidal antiandrogen drug and/or a steroidal antiandrogen.

7. A composition comprising minoxidil for use in a method for the treatment of hair loss or excessive hair shedding in a subject or for promoting hair growth in a subject according to any one of the claims 1 to 6, which further comprises administering spironolactone within the range of from 10 mg to 500 mg, or from 10 mg to 400 mg, or from 10 mg to 300 mg, or from 15 mg to 200 mg, or from 15 mg to 150 mg, or from 18 mg to 100 mg, or from 20 mg to 80 mg, or from 20 mg to 50 mg, or from 22 mg to 40 mg, or from 23 mg to 35 mg, or from 23 mg to 30 mg, or is 25 mg.

8. A composition comprising minoxidil for use in a method for the treatment of hair loss or excessive hair shedding in a subject or for promoting hair growth in a subject according to any one of the claims 1 to 7, which further comprises administering sodium chloride within the range of from 10 mg to 200 mg, or from 15 mg to 150 mg, or from 15 mg to 125 mg, or from 20 mg to 100 mg, or from 25 mg to 80 mg, or from 30 mg to 70 mg, or from 40 mg to 60 mg, or from 45 mg to 55 mg, or is 50 mg, or is 20 mg.

9. A composition comprising minoxidil for use in a method for the treatment of hair loss or excessive hair shedding in a subject or for promoting hair growth in a subject according to any one of the claims 1 to 8, which additionally comprises administering one or more of:
(i) finasteride within the range of from 0.1 mg to 1 mg;
(ii) dutasteride within the range of from 0.01 mg to 1 mg;
(iii) flutamide within the range of from 10 mg to 500 mg;
(iv) cyproterone acetate within the range of from 1 mg to 100 mg;
(v) bicalutamide within the range of from 1 mg to 100 mg;
(vi) enzalutamide within the range of from 1 mg to 100 mg;
(vii) nilutamide within the range of from 1 mg to 100 mg;
(viii) drosperidone within the range of from 0.1 mg to 10 mg;
(ix) apalutamide within the range of from 1 mg to 100 mg; and/or
(x) buseralin within the range of from 0.1 mg to 10 mg.

10. A composition comprising minoxidil for use in a method for the treatment of hair loss or excessive hair shedding in a subject or for promoting hair growth in a subject according to any one of the claims 1 or 2, wherein
a) the hair loss or excessive hair shedding is the result of one or more of the following; hair follicle miniaturization, alopecia areata, androgenetic alopecia, telogen effluvium, anagen effluvium, chemotherapy induced hair loss, male pattern baldness, female pattern baldness, monilethrix, thyroid problems, anaemia, polycystic ovary syndrome, cicatricial alopecia (lichen planopilaris, discoid lupus erythematosus, folliculitis decalvans), congenital hypotrichosis, loose anagen hair syndrome, hypotrichosis and malnutrition; and/or
b)promoting hair growth comprises promoting beard growth in a subject; and/or
c) promoting hair growth comprises increasing hair length.

11. An orodispersible composition for treating hair loss or excessive hair shedding in a subject or for promoting hair growth in a subject comprising;
(i) minoxidil within the range of from 0.05 mg to 3 mg;
(ii) minoxidil at a concentration of 0.1 mg;
(iii) minoxidil at a concentration of 0.15 mg;
(iv) minoxidil at a concentration of 0.24 mg;
(v) minoxidil at a concentration of 0.25 mg;
(vi) minoxidil at a concentration of 0.45 mg;
(vi) minoxidil at a concentration of 1.35 mg;
(vii) minoxidil within the range of from 0.05 mg to 3 mg and spironolactone within the range of from 10 mg to 500 mg; or
(viii) minoxidil within the range of from 0.15 mg to 1.35 mg and spironolactone within the range of from 10 mg to 500 mg.

12. The composition according to claim 11, wherein the composition is in a form selected from: strip, wafer, pellet, film, troche, tablet, lipid matrix tablet, capsule, pill, granule, pellet, powder, drop, spray and lozenge.

13. The composition according to any one of the claims 11 or 12, wherein the composition comprises:
(i) a disintegration agent which aids disintegration of the composition in the presence of saliva; and/or
(ii) an agent which can be acted upon by an enzyme in saliva to facilitate disintegration.

14. The composition according to any one of claims 11 to 13, wherein the composition comprises a taste modifying agent.

## Patentansprüche

1. Zusammensetzung umfassend Minoxidil zur Verwendung in einem Verfahren zur Behandlung von Haarausfall oder übermäßigem Haarverlust bei einer Person oder zur Förderung des Haarwachstums bei einer Person durch Verabreichung einer wirksamen Dosis von Minoxidil an eine Person durch die sublinguale Schleimhaut.

2. Zusammensetzung umfassend Minoxidil zur Verwendung in einem Verfahren zur Behandlung von Haarausfall oder übermäßigem Haarverlust bei einer Person oder zur Förderung des Haarwachstums bei einer Person gemäß Anspruch 1, wobei die Dosis von Minoxidil in einer Form vorliegt, die sich in Gegenwart von Speichel auflöst.

3. Zusammensetzung umfassend Minoxidil zur Verwendung in einem Verfahren zur Behandlung von Haarausfall oder übermäßigem Haarverlust bei einer Person oder zur Förderung des Haarwachstums bei einer Person gemäß einem der Ansprüche 1 oder 2, wobei die Dosis von Minoxidil
a) in einer Minoxidil-Blutkonzentration von 0,25 ng/ml bis 10 ng/ml resultiert; und/oder
b) im Bereich von 0,05 mg bis 3 mg, oder von 0,1 mg bis 2,5 mg, oder von 0,15 mg bis 2 mg, oder von 0,15 mg bis 1,5 mg, oder von 0,15 mg bis 1 mg, oder von 0.15 mg bis 0,8 mg, oder von 0,1 mg bis 0,5 mg, oder von 0,5 mg bis 0,25 mg, oder von 0,1 mg, oder von 0,15 mg, oder von 0,24 mg, oder von 0,25 mg, oder von 0,45 mg, oder von 1,35 mg ist.

4. Zusammensetzung umfassend Minoxidil zur Verwendung in einem Verfahren zur Behandlung von Haarausfall oder übermäßigem Haarverlust bei einer Person oder zur Förderung des Haarwachstums bei einer Person gemäß einem der Ansprüche 1 bis 3, wobei die Dosis von Minoxidil mindestens alle 3 Tage, mindestens alle 2 Tage oder mindestens täglich verabreicht wird.

5. Zusammensetzung umfassend Minoxidil zur Verwendung in einem Verfahren zur Behandlung von Haarausfall oder übermäßigem Haarverlust bei einer Person oder zur Förderung des Haarwachstums bei einer Person gemäß einem der Ansprüche 1 bis 4, wobei die Dosis von Minoxidil in einer Form vorliegt, die ausgewählt ist aus: Streifen, Plättchen, Pellet, Film, Troche, Tablette, Lipidmatrixtablette, Kapsel, Pille, Granulat, Pellet, Pulver, Tropfen, Spray und Lutschtablette.

6. Zusammensetzung umfassend Minoxidil zur Verwendung in einem Verfahren zur Behandlung von Haarausfall oder übermäßigem Haarverlust bei einer Person oder zur Förderung des Haarwachstums bei einer Person gemäß einem der Ansprüche 1 bis 5, welches ferner die Verabreichung eines Aldosteronantagonisten, 5a-Reduktaseinhibitors, nichtsteroidalen Antiandrogens und/oder eines steroidalen Antiandrogens umfasst.

7. Zusammensetzung umfassend Minoxidil zur Verwendung in einem Verfahren zur Behandlung von Haarausfall oder übermäßigem Haaraverlust bei einer Person oder zur Förderung des Haarwachstums bei einer Person gemäß einem der Ansprüche 1 bis 6, welches ferner die Verabreichung von Spironolacton im Bereich von 10 mg bis 500 mg, oder von 10 mg bis 400 mg, oder von 10 mg bis 300 mg, oder von 15 mg bis 200 mg, oder von 15 mg bis 150 mg, oder von 18 mg bis 100 mg, oder von 20 mg bis 80 mg, oder von 20 mg bis 50 mg, oder von 22 mg bis 40 mg, oder von 23 mg bis 35 mg, oder von 23 mg bis 30 mg, oder 25 mg umfasst.

8. Zusammensetzung umfassend Minoxidil zur Verwendung in einem Verfahren zur Behandlung von Haarausfall oder übermäßigem Haarverlust bei einer Person oder zur Förderung des Haarwachstums bei einer Person nach einem der Ansprüche 1 bis 7, die ferner die Verabreichung von Natriumchlorid im Bereich von 10 mg bis 200 mg, oder von 15 mg bis 150 mg, oder von 15 mg bis 125 mg, oder von 20 mg bis 100 mg, oder von 25 mg bis 80 mg, oder von 30 mg bis 70 mg, oder von 40 mg bis 60 mg, oder von 45 mg bis 55 mg, oder von 50 mg, oder von 20 mg umfasst.

9. Zusammensetzung umfassend Minoxidil zur Verwendung in einem Verfahren zur Behandlung von Haarausfall oder übermäßigem Haarverlust bei einer Person oder zur Förderung des Haarwachstums bei einer Person gemäß einem der Ansprüche 1 bis 8, das zusätzlich die Verabreichung eines oder mehrerer der folgenden Mittel umfasst:
(i) Finasterid in einem Bereich von 0,1 mg bis 1 mg;
(ii) Dutasterid in einem Bereich von 0,01 mg bis 1 mg;
(iii) Flutamid in einem Bereich von 10 mg bis 500 mg;
(iv) Cyproteronacetat in einem Bereich von 1 mg bis 100 mg;
(v) Bicalutamid in einem Bereich von 1 mg bis 100 mg;
(vi) Enzalutamid in einem Bereich von 1 mg bis 100 mg;
(vii) Nilutamid in einem Bereich von 1 mg bis 100 mg;
(viii) Drosperidon in einem Bereich von 0,1 mg bis 10 mg;
(ix) Apalutamid in einem Bereich von 1 mg bis 100 mg; und/oder
(x) Buseralin in einem Bereich von 0,1 mg bis 10 mg.

10. Zusammensetzung umfassend Minoxidil zur Verwendung in einem Verfahren zur Behandlung von Haarausfall oder übermäßigem Haarverlust bei einer Person oder zur Förderung des Haarwachstums bei einer Person gemäß einem der Ansprüche 1 oder 2, wobei
a) der Haarausfall oder übermäßige Haarverlust das Ergebnis einer oder mehrerer der folgenden Ursachen ist: Miniaturisierung der Haarfollikel, Alopecia areata, androgenetische Alopezie, telogenes Effluvium, anagenes Effluvium, Chemotherapie-bedingter Haarausfall, Kahlköpfigkeit bei Männern, Kahlköpfigkeit bei Frauen, Monilethrix, Schilddrüsenprobleme, Anämie, polyzystisches Ovarialsyndrom, narbige Alopezie (Lichen planopilaris, diskoider Lupus erythematodes, Follikulitis decalvans), angeborene Hypotrichose, Syndrom des schütteren anagenen Haares, Hypotrichose und Mangelernährung; und/oder
b) die Förderung des Haarwuchses die Förderung des Bartwuchses bei einer Person umfasst; und/oder
c) die Förderung des Haarwachstums die Erhöhung der Haarlänge umfasst.

11. Orodispersible Zusammensetzung zur Behandlung von Haarausfall oder übermäßigem Haarverlust bei einer Person oder zur Förderung des Haarwachstums bei einer Person, umfassend;
(i) Minoxidil im Bereich von 0,05 mg bis 3 mg;
(ii) Minoxidil in einer Konzentration von 0,1 mg;
(iii) Minoxidil in einer Konzentration von 0,15 mg;
(iv) Minoxidil in einer Konzentration von 0,24 mg;
(v) Minoxidil in einer Konzentration von 0,25 mg;
(vi) Minoxidil in einer Konzentration von 0,45 mg;
(vi) Minoxidil in einer Konzentration von 1,35 mg;
(vii) Minoxidil im Bereich von 0,05 mg bis 3 mg und Spironolacton im Bereich von 10 mg bis 500 mg; oder
(viii) Minoxidil in einem Bereich von 0,15 mg bis 1,35 mg und Spironolacton in einem Bereich von 10 mg bis 500 mg.

12. Zusammensetzung gemäß Anspruch 11, wobei die Zusammensetzung in einer Form vorliegt, die ausgewählt ist aus: Streifen, Plättchen, Pellet, Film, Troche, Tablette, Lipidmatrixtablette, Kapsel, Pille, Granulat, Pellet, Pulver, Tropfen, Spray und Lutschtablette.

13. Zusammensetzung gemäß einem der Ansprüche 11 oder 12, wobei die Zusammensetzung umfasst:
(i) ein Sprengmittel, das den Zerfall der Zusammensetzung in Gegenwart von Speichel unterstützt; und/oder
(ii) ein Mittel, auf das ein Enzym im Speichel einwirken kann, um den Zerfall zu erleichtern.

14. Zusammensetzung gemäß einem der Ansprüche 11 bis 13, wobei die Zusammensetzung ein geschmacksveränderndes Mittel enthält.

## Revendications

1. Une composition comprenant du minoxidil pour une utilisation dans une méthode de traitement de la perte des cheveux ou de la chute excessive des cheveux chez un sujet ou pour favoriser la pousse des cheveux chez un sujet en administrant à un sujet une dose efficace de minoxidil à travers la muqueuse sublinguale.

2. Une composition comprenant du minoxidil pour une utilisation dans une méthode de traitement de la perte des cheveux ou de la chute excessive des cheveux chez un sujet ou pour favoriser la pousse des cheveux chez un sujet, selon la revendication 1, dans laquelle la dose de minoxidil est sous une forme qui se désagrège en présence de salive.

3. Une composition comprenant du minoxidil pour une utilisation dans une méthode de traitement de la perte des cheveux ou de la chute excessive des cheveux chez un sujet ou pour favoriser la pousse des cheveux chez un sujet, selon l'une quelconque des revendications 1 ou 2, dans laquelle la dose de minoxidil
a) entraîne une concentration de minoxidil dans le sang allant de 0,25 ng/ml à 10 ng/ml ; et/ou
b) se situe dans la gamme allant de 0,05 mg à 3 mg, ou de 0,1 mg à 2,5 mg, ou de 0,15 mg à 2 mg, ou de 0,15 mg à 1,5 mg, ou de 0,15 mg à 1 mg, ou de 0,15 mg à 0,8 mg, ou de 0,15 mg à 0,5 mg, ou de 0,5 mg à 0,25 mg, ou est de 0,1 mg, ou est de 0,15 mg, ou est de 0,24 mg, ou est de 0,25 mg, ou est de 0,45 mg, ou est de 1,35 mg.

4. Une composition comprenant du minoxidil pour une utilisation dans une méthode de traitement de la perte des cheveux ou de la chute excessive des cheveux chez un sujet ou pour favoriser la pousse des cheveux chez un sujet, selon l'une quelconque des revendications 1 à 3, dans laquelle la dose de minoxidil est administrée au moins tous les 3 jours, au moins tous les 2 jours, ou au moins tous les jours.

5. Une composition comprenant du minoxidil pour une utilisation dans une méthode de traitement de la perte des cheveux ou de la chute excessive des cheveux chez un sujet ou pour favoriser la pousse des cheveux chez un sujet, selon l'une quelconque des revendications 1 à 4, dans laquelle la dose de minoxidil est en une forme choisie parmi : une bande, une plaquette, une pastille, un film, un cachet, un comprimé, un comprimé à matrice lipidique, une gélule, une pilule, un granule, une pastille, une poudre, une goutte, un spray et une pastille en losange.

6. Une composition comprenant du minoxidil pour une utilisation dans une méthode de traitement de la perte des cheveux ou de la chute excessive des cheveux chez un sujet ou pour favoriser la pousse des cheveux chez un sujet, selon l'une quelconque des revendications 1 à 5, qui comprend en outre le fait d'administrer : un antagoniste de l'aldostérone, un inhibiteur de la 5α-réductase, un médicament anti-androgène non stéroïdien et/ou un anti-androgène stéroïdien.

7. Une composition comprenant du minoxidil pour une utilisation dans une méthode pour le traitement de la perte des cheveux ou de la chute excessive des cheveux chez un sujet ou pour favoriser la pousse des cheveux chez un sujet, selon l'une quelconque des revendications 1 à 6, qui comprend en outre le fait d'administrer de la spironolactone dans une gamme allant de 10 mg à 500 mg, ou de 10 mg à 400 mg, ou de 10 mg à 300 mg, ou de 15 mg à 200 mg, ou de 15 mg à 150 mg, ou de 18 mg à 100 mg, ou de 20 mg à 80 mg, ou de 20 mg à 50 mg, ou de 22 mg à 40 mg, ou de 23 mg à 35 mg, ou de 23 mg à 30 mg, ou en une quantité de 25 mg.

8. Une composition comprenant du minoxidil pour une utilisation dans une méthode de traitement de la perte des cheveux ou de la chute excessive des cheveux chez un sujet ou pour favoriser la pousse des cheveux chez un sujet, selon l'une quelconque des revendications 1 à 7, qui comprend en outre le fait d'administrer du chlorure de sodium dans une gamme allant de 10 mg à 200 mg, ou de 15 mg à 150 mg, ou de 15 mg à 125 mg, ou de 20 mg à 100 mg, ou de 25 mg à 80 mg, ou de 30 mg à 70 mg, ou de 40 mg à 60 mg, ou de 45 mg à 55 mg, ou est de 50 mg, ou en une quantité de 20 mg.

9. Une composition comprenant du minoxidil pour une utilisation dans une méthode de traitement de la perte des cheveux ou de la chute excessive des cheveux chez un sujet ou pour favoriser la pousse des cheveux chez un sujet, selon l'une quelconque des revendications 1 à 8, qui comprend en outre le fait d'administrer un ou plusieurs parmi :
(i) du finastéride dans la gamme allant de 0,1 mg à 1 mg ;
(ii) du dutastéride dans la gamme allant de 0,01 mg à 1 mg ;
(iii) du flutamide dans la gamme allant de 10 mg à 500 mg ;
(iv) de l'acétate de cyprotérone dans la gamme allant de 1 mg à 100 mg ;
(v) du bicalutamide dans la gamme allant de 1 mg à 100 mg ;
(vi) de l'enzalutamide dans la gamme allant de 1 mg à 100 mg ;
(vii) du nilutamide dans la gamme allant de 1 mg à 100 mg ;
(viii) de la drospéridone dans la gamme allant de 0,1 mg à 10 mg ;
(ix) de apalutamide dans la gamme allant de 1 mg à 100 mg ; et/ou
(x) du buséraline dans la gamme allant de 0,1 mg à 10 mg.

10. Une composition comprenant du minoxidil pour une utilisation dans une méthode de traitement de la perte des cheveux ou de la chute excessive des cheveux chez un sujet ou pour favoriser la pousse des cheveux chez un sujet, selon l'une quelconque des revendications 1 ou 2, dans laquelle
a) la perte des cheveux ou la chute excessive des cheveux est le résultat d'un ou plusieurs des éléments suivants : miniaturisation du follicule pileux, alopécie en zone, alopécie androgénétique, effluve télogène, effluve anagène, perte de cheveux induite par la chimiothérapie, calvitie masculine, calvitie féminine, monilethrix, problèmes thyroïdiens, anémie, syndrome des ovaires polykystiques, alopécie cicatricielle (lichen planopilaris, lupus érythémateux discoïde, folliculite décalvanique), hypotrichose congénitale, syndrome des cheveux anagènes lâches, hypotrichose et malnutrition ; et/ou
b) le fait de favoriser la pousse des cheveux comprend le fait de favoriser la pousse de la barbe chez un sujet ; et/ou
c) le fait de favoriser la pousse des cheveux comprend le fait d'augmenter la longueur des cheveux.

11. Une composition orodispersible pour traiter la perte des cheveux ou la chute excessive des cheveux chez un sujet ou pour favoriser la pousse des cheveux chez un sujet comprenant ;
(i) du minoxidil dans la gamme allant de 0,05 mg à 3 mg ;
(ii) du minoxidil à une concentration de 0,1 mg ;
(iii) du minoxidil à une concentration de 0,15 mg ;
(iv) du minoxidil à une concentration de 0,24 mg ;
(v) du minoxidil à une concentration de 0,25 mg ;
(vi) du minoxidil à une concentration de 0,45 mg ;
(vii) du minoxidil à une concentration de 1,35 mg ;
(vii) du minoxidil dans la gamme allant de 0,05 mg à 3 mg et de la spironolactone dans la gamme allant de 10 mg à 500 mg ; ou
(viii) du minoxidil dans la gamme allant de 0,15 mg à 1,35 mg et de la spironolactone dans la gamme allant de 10 mg à 500 mg.

12. La composition selon la revendication 11, se présentant sous une forme choisie parmi : une bande, une plaquette, une pastille, un film, un cachet, un comprimé, un comprimé à matrice lipidique, une gélule, une pilule, un granule, une pastille, une poudre, une goutte, un spray et pastille en losange.

13. La composition selon l'une quelconque des revendications 11 ou 12, dans laquelle la composition comprend :
(i) un agent de désintégration qui facilite la désintégration de la composition en présence de salive ; et/ou
(ii) un agent sur lequel peut agir une enzyme dans la salive pour faciliter la désintégration.

14. Une composition selon l'une quelconque des revendications 11 à 13, dans laquelle la composition comprend un agent modifiant le goût.
